(19) Europäisches Patentamt
European Patent Office
Office européen des brevets

(11) EP 3 601 292 B1

(12) **FASCICULE DE BREVET EUROPEEN**

(45) Date de publication et mention de la délivrance du brevet:
**11.09.2024 Bulletin 2024/37**

(21) Numéro de dépôt: **18718883.4**

(22) Date de dépôt: **27.03.2018**

(51) Classification Internationale des Brevets (IPC):
***C07D 491/18*** (2006.01) ***C08F 265/06*** (2006.01)
***C08F 222/00*** (2006.01) ***C08L 33/12*** (2006.01)

(52) Classification Coopérative des Brevets (CPC):
(C-Sets disponibles)
**C07D 491/18; C08F 265/06** (Cont.)

(86) Numéro de dépôt international:
**PCT/FR2018/050736**

(87) Numéro de publication internationale:
**WO 2018/178556 (04.10.2018 Gazette 2018/40)**

(54) **COMPOSE RETICULANT, SON PROCEDE DE SYNTHESE, COMPOSITION LIQUIDE COMPRENANT LEDIT COMPOSE RETICULANT, SON PROCEDE DE POLYMERISATION, ET MATERIAU OBTENU APRES POLYMERISATION DE LADITE COMPOSITION**

VERNETZENDE VERBINDUNG, VERFAHREN ZU IHRER SYNTHETISIERUNG, FLÜSSIGE ZUSAMMENSETZUNG MIT DIESER VERNETZENDEN VERBINDUNG, VERFAHREN ZU IHRER POLYMERISATION UND NACH DER POLYMERISATION DIESER ZUSAMMENSETZUNG ERHALTENES MATERIAL

CROSSLINKING COMPOUND, METHOD FOR SYNTHESIZING SAME, LIQUID COMPOSITION COMPRISING SAID CROSSLINKING COMPOUND, PROCESS FOR POLYMERIZING SAME, AND MATERIAL OBTAINED AFTER POLYMERIZATION OF SAID COMPOSITION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.03.2017 FR 1752544**

(43) Date de publication de la demande:
**05.02.2020 Bulletin 2020/06**

(73) Titulaire: **Arkema France**
**92700 Colombes (FR)**

(72) Inventeurs:
- **FONTANIER, Jean-Charles**
**81000 Albi (FR)**
- **GERARD, Jean-François**
**69500 Bron (FR)**
- **GERARD, Pierre**
**64230 Denguin (FR)**
- **LORTIE, Frédéric**
**69100 Villeurbanne (FR)**
- **PASCAULT, Jean-Pierre**
**69100 Villeurbanne (FR)**

(74) Mandataire: **Arkema Patent**
**Arkema France**
**DRD-DPI**
**420, rue d'Estienne d'Orves**
**92705 Colombes Cedex (FR)**

(56) Documents cités:
**CN-A- 105 294 936** **CN-A- 105 294 936**
**GB-A- 2 453 112** **JP-A- 2005 232 412**
**JP-A- 2014 169 384** **US-A1- 2014 329 929**

- **HAO SUN ET AL: "Thermally-labile segmented hyperbranched copolymers: using reversible-covalent chemistry to investigate the mechanism of self-condensing vinyl copolymerization", CHEMICAL SCIENCE, vol. 5, no. 12, 1 January 2014 (2014-01-01), United Kingdom, pages 4646 - 4655, XP055405239, ISSN: 2041-6520, DOI: 10.1039/C4SC02290D**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



**(Cont. page suivante)**

(52) Classification Coopérative des Brevets (CPC): (Cont.)

C-Sets
**C08F 265/06, C08F 220/14, C08F 222/102**

## Description

L'invention concerne le domaine des polymères thermoplastiques et plus particulièrement des matériaux composites comprenant des polymères thermoplastiques. La présente invention concerne une composition liquide comprenant un monomère, un polymère (méth)acrylique et un composé réticulant. Cette composition liquide peut être utilisée sous la forme d'un sirop en particulier pour l'imprégnation de fibres ou de matériau fibreux. L'invention concerne en outre un matériau thermoplastique obtenu après polymérisation de la composition liquide. L'invention concerne en outre un procédé d'imprégnation d'un substrat fibreux de fibres longues avec ladite composition liquide. L'invention concerne en outre un substrat fibreux imprégné avec ladite composition liquide qui est utile pour fabriquer des pièces composites. La présente invention concerne également un procédé de fabrication de pièces mécaniques ou d'éléments structuraux constitués de matériau composite et de pièces mécaniques obtenus par un procédé utilisant une telle composition liquide.

### [Art antérieur]

Les polymères thermoplastiques sont des matériaux qui sont largement utilisés aujourd'hui dans plusieurs domaines tels que les secteurs de la construction, l'aéronautique, l'automobile ou les chemins de fer, dans lesquels ils font partie de pièces mécaniques. Ces pièces mécaniques, qui comportent des polymères thermoplastiques, peuvent supporter des contraintes élevées, chimiques ou mécaniques, pendant leur utilisation et sont généralement fabriquées à partir de matériaux composites. Un matériau composite est une combinaison macroscopique de deux ou plus de deux matériaux non miscibles. Les matériaux composites sont généralement constitués d'au moins un matériau qui forme la matrice, c'est-à-dire une phase continue qui assure la cohésion de la structure, et d'un matériau de renforcement. L'utilisation d'un matériau composite permet d'obtenir des performances qui ne sont pas disponibles à partir de chacun de ses constituants lorsqu'ils sont utilisés séparément. Par conséquent, et notamment en raison de leurs meilleures performances mécaniques (résistance à la traction plus élevée, module de traction plus élevée, ténacité plus élevée) et leur faible masse volumique par rapport à des matériaux homogènes, les matériaux composites sont largement utilisés dans les secteurs industriels tels que le bâtiment, l'automobile, l'aérospatiale, le transport, les loisirs, l'électronique et les sports.

Dans ces secteurs, les matériaux composites utilisés sont souvent à base de polymères thermodurcissables car ces derniers présentent des propriétés mécaniques intéressantes et des caractéristiques physicochimiques, telles qu'une résistance aux solvants, souhaitables dans de nombreux domaines d'applications.

Néanmoins, afin de permettre le thermoformage et le recyclage, il est préférable d'utiliser, dans les matériaux composites, des polymères thermoplastiques. Les polymères thermoplastiques sont constitués de polymères linéaires ou ramifiés qui ne sont généralement pas réticulés. Par exemple, une composition liquide, ou un sirop, comprenant un monomère (méth)acrylique et un polymère (méth)acrylique est décrit dans WO 2013/056845, WO 2014/013028 et WO2014/174098.

Le document GB2453112 décrit un agent viscosifiant apte à subir une réaction de Diels-Alder, composé d'un polymère et d'un agent réticulant.

Le document JP2014169384 décrit un procédé de formation d'une résine présentant des propriétés de flexion, pour former des surfaces incurvées, comportant l'utilisation d'un monomère possédant un adduit Diels Alder (formé à partir d'un furane et d'un maléimide) et au moins un groupement de type acryloyl aussi appelé prop-2-enoyl.

Le document JP2005232412 décrit un procédé de synthèse d'un polymère réticulé présentant des liaisons de réticulation à température ambiante pouvant se dissocier à température élevée.

Cependant, les matériaux comportant des polymères thermoplastiques peuvent présenter une certaine sensibilité aux solvants par rapport aux matériaux thermodurcissables. Ainsi, depuis quelques années, de nombreuses études sur le développement de réseaux réversibles ont été menées en se basant notamment sur les réactions Diels-Alder (DA) et rétro Diels-Alder (rDA). En effet, outre le large domaine d'applications possibles, ces réseaux dits « réversibles » sont très intéressants car ils permettent de passer aisément d'un polymère tridimensionnel à un polymère linéaire. Ce type de réseau est par exemple obtenu par l'addition d'un diène conjugué sur un diènophile.

Les stratégies proposées consistent généralement à piloter la formation du réseau par réaction DA en faisant réagir un diènophile sur un diène conjugué incorporé dans une chaîne polymère ou encore en faisant réagir ensemble un couple de monomères multifonctionnels diènes/diènophiles de façon à former le polymère réticulé. Ainsi, comme cela est détaillé dans le document WO2010033028, un copolymère portant des fonctions furanes pendantes qui en présence d'un bisdiènophile, à une température adéquate, permet par réaction DA de ponter deux chaînes polymères entre elles, conduisant ainsi à l'obtention d'un réseau tridimensionnel. Ensuite, chauffée à une température adéquate, la réaction d'addition DA devient réversible et permet de retrouver les produits initiaux. Dans ledit document, le temps de réticulation le plus rapide était d'environ 2 heures. Ainsi, cette technique n'est pas adaptée à une utilisation industrielle. En effet, la cinétique de cette réaction est trop lente et le taux de conversion est faible.

En outre, cette absence de conversion complète conduit à la présence dans le matériau polymère final d'une

portion de composés diénophile ou diène conjugué n'ayant pas réagi et pouvant conduire à une réactivité ultérieure non maîtrisée.

Ainsi, il existe un besoin pour de nouveaux composés réticulant et compositions liquides pour la formation de polymères thermoplastiques capables de répondre aux problèmes engendrés par les méthodes existantes.

**[Problème technique]**

Un objectif de la présente invention est de disposer d'un composé réticulant capable d'améliorer les propriétés mécaniques et physicochimiques des polymères thermoplastiques. Un autre objectif de la présente invention est de disposer d'une composition liquide comprenant un monomère, un polymère (méth)acrylique et un composé réticulant pour obtenir une composition qui peut être utilisée dans des procédés industriels nécessitant des cycles de production courts, par exemple inférieurs à cinq minutes.

Un objectif de la présente invention est également de disposer d'un procédé de formation d'un polymère thermoplastique au moins partiellement réticulé avec une bonne conversion c'est-à-dire qui comporte moins de 5 % de fonctions diènes conjugués et diènophiles, aptes à subir une réaction d'addition de Diels-Alder, libres, c'est-à-dire n'ayant pas fait l'objet d'une réaction d'addition de Diels-Alder.

Un autre objet de la présente invention est de proposer un procédé qui peut être mis en oeuvre à un coût faible et qui permet la fabrication à l'échelle industrielle de pièces mécaniques ou éléments structuraux constitués de polymère thermoplastique au moins partiellement réticulé ou de matériau composite thermoplastique au moins partiellement réticulé. La fabrication des pièces composites doit également être reproductible et rapide, ce qui signifie des temps de cycle courts.

**[Brève description de l'invention]**

A cet effet, l'invention porte sur une composition liquide comprenant :

a. au moins 5 % en poids et pas plus de 50 % en poids d'un polymère (méth)acrylique (P1),
b. entre 40 % et 90 % en poids d'un monomère (méth)acrylique (M1) ou un mélange de monomères (méth)acryliques, et
c. supérieure à 0,5 phr et inférieure à 10 phr, par rapport à la somme du monomère (méth)acrylique (M1) et du polymère (méth)acrylique (P1), d'un composé réticulant de formule (I)

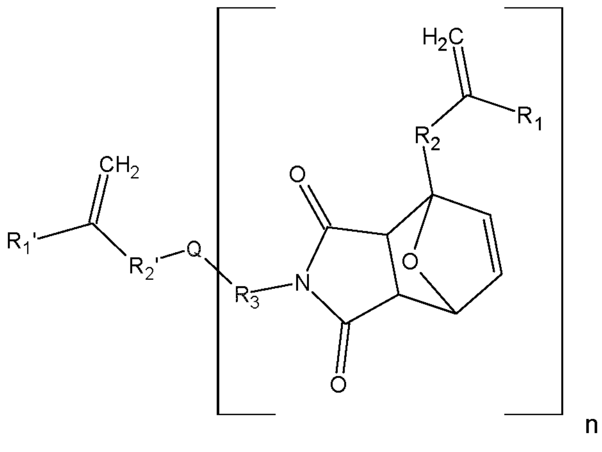

(I)

dans laquelle :

le groupement Q représente -Z-L- où

le groupement Z représente une simple liaison ou

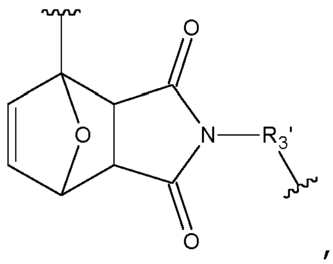

le groupement L représente une simple liaison ou un groupe sélectionné parmi : - $(C_1-C_6)$alkyle-, - $(C_2-C_6)$alkényle-, -$(C_2-C_6)$alkynyle-, -$(CONHR_4NHCOOR_5O)_m$-, -$(O-CO-NR_6)_m$-, -$(ORCHOHR_5)_m$-, - $(CH_2CHOHCH_2OR_4O)_m$-, -$(OR_4)_m$-, -$((CHOR_6)CH_2O-R_4)_m$-, -$(R_4-COO-R_5)_m$-, - $NR_6$-, -$SO_2$-, -$SO_2NR_6$-, -O-, -S-, -$CONR_6$-, -aryle-, -(aryle-$R_4)_m$-, -(hétéroaryle-$R_4)_m$-, -(($C_3-C_8$)hétérocyclyle-$R_4)_m$-, -(($C_3-C_{14}$)cycloalkyle-$R_4)_m$-, - $(C_1-C_6)$alkyle-$R_7$-$(C_1-C_6)$alkyle-, -$(C_2-C_6)$alkényle-$R_7$-$(C_1-C_6)$alkyle-, - $(C_1-C_6)$alkyle-$R_7$-$(C_2-C_6)$alkényle-, ou - $(C_2-C_6)$alkényle-$R_7$-$(C_2-C_6)$alkényle-,

où les groupes $R_4$ et $R_5$ sont identiques ou différents et représentent un groupe sélectionné parmi : simple liaison, -$(C_1-C_6)$alkyle-, -$(C_2-C_6)$alkényle-, -$NR_6$-, -$SO_2$-, -$SO_2NR_6$-, -O-, -S-, -$CONR_6$-, -aryle-, -(aryle-$R_6)_m$-, - (hétéroaryle-$R_6)_m$-, - (($C_3-C_8$)hétérocyclyle)-$R_6)_m$-, - (($C_3-C_{14}$)cycloalkyle-$R_6)_m$-aryle-, -hétéroaryle-, -$(C_3-C_8)$ hétérocyclyle-, -$(C_3-C_{14})$cycloalkyle-, -$(C_1-C_6)$alkyle-OCO-, ou -$CH_2$-($CHOR_3$)$CH_2O$-$(C_1-C_6)$alkyle- ;
où $R_6$ représente un groupe sélectionné parmi : atome d'hydrogène, - (Ci-Ce) alkyle, - $(C_2-C_6)$alkényle, -$NH_2$, -COOH, -$SO_2H$, -OH, -SH, -aryle, -hétéroaryle, - $(C_3-C_8)$hétérocyclyle, ou - $(C_3-C_{14})$cycloalkyle ;
où $R_7$ représente un groupe sélectionné parmi : simple liaison, - $(CONHR_4NHCOOR_5O)_m$-, - (O-CO-NR4$)_m$-, - $(COR_4)_m$-, - $(OR_4)_m$-, - $(CH_2CHOHCH_2OR_4O)_m$-, - $((CHOR_4)CH_2O-R_5)_m$-, - $(R_4-COO-R_5)_m$-, -$NR_6$-, -$SO_2$-, -$SO_2NR_6$-, -O-, -S-, -$CONR_6$-, -aryle-, -(aryle-$R_4)_m$-, - (hétéroaryle-$R_4)_m$-, -(($C_3-C_8$)hétérocyclyle) -$R_4)_m$-, - (($C_3-C_{14}$)cycloalkyle-$R_4)_m$-, - $(C_1-C_6)$alkyle-OCO-, ou -$CH_2$-($CHOR_3$)$CH_2O$-$(C_1-C_6)$alkyle- ;

les groupements $R_1$ et $R_1$' sont identiques ou différents et représentent un atome d'hydrogène ou un groupe alkyle à chaine linéaire ou ramifiée contenant jusqu'à 6 atomes de carbone ; les groupements $R_2$ et $R_2$' sont identiques ou différents et représentent une simple liaison ou un groupe sélectionné parmi : - (Ci-Ce) alkyle-, - $(C_2-C_6)$alkényle-, -COO-, ou -COO- (Ci-Ce) alkyle-, de préférence -COO- ou -COO-$(C_1-C_6)$alkyle- ; les groupements $R_3$ et $R_3$' sont identiques ou différents et représentent un groupe sélectionné parmi : -NHCOO- ;

avec n, représentant le nombre d'unités de répétition, compris entre 1 et 10,
avec m, représentant le nombre d'unités de répétition, compris entre 1 et 20, par exemple entre 1 et 10.

Le composé réticulant comporte deux fonctions vinyliques polymérisables et au moins un produit d'addition de Diels-Alder correspondant à un groupement apte à subir une réaction de rétro Diels-Alder.

Le choix du groupement « L » permet de faire varier les propriétés du composé réticulant et plus largement d'un polymère pseudo thermoplastique pouvant être obtenu à partir dudit composé réticulant. En effet, l'espaceur « L » apporte des propriétés différentes au réseau final. Ainsi, un espaceur « L » présentant une longue chaîne carbonée entre ses deux fonctions maléimides permet la formation d'un réseau souple et thermiquement peu résistant. A l'inverse, une structure courte présentant un ou plusieurs noyaux aromatiques sera à l'origine d'un réseau rigide et thermiquement résistant.

Comme cela sera présenté dans la suite de la demande, ce composé réticulant peut comporter un seul groupement de type époxyisoindole, issue de l'addition de Diels-Alder, ou bien plusieurs de ces groupements de type époxyisoindole.

L'invention porte en outre sur composition liquide dans laquelle la procédé de synthèse d'un composé réticulant caractérisé en ce qu'il comprend une étape de réaction d'addition Diels-Alder entre un maléimide de formule (II)

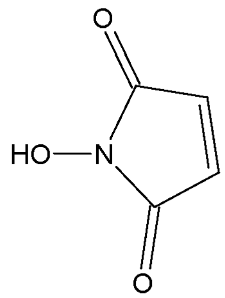

(II)

et une molécule de formule (III)

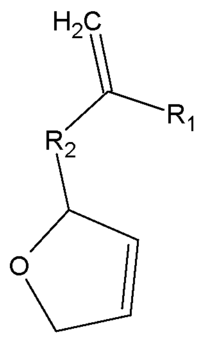

(III)

dans laquelle :

le groupement $R_1$ représente un atome d'hydrogène ou un groupe alkyle à chaine linéaire, ou ramifiée contenant jusqu'à 6 atomes de carbone ;

le groupement $R_2$ représente une simple liaison ou un groupe sélectionné parmi : $-(C_1-C_6)$alkyle-, $-(C_2-C_6)$alkényle-, -COO-, ou $-(C_1-C_6)$alkyle-COO-, de préférence -COO- ou $-(C_1-C_6)$alkyle-COO- ;

de façon à former un produit d'addition Diels-Alder ;
et une étape de réaction entre ledit produit d'addition Diels-Alder avec une molécule comportant au moins une fonction isocyanate sélectionnée parmi les molécules de formule (IV) ou de formule (V)

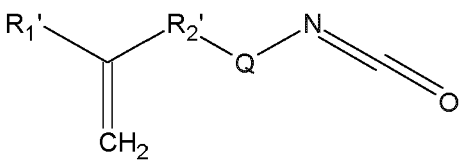

(IV)

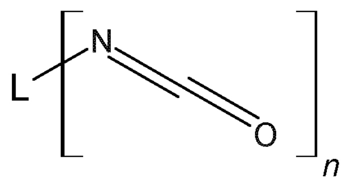

(V)

dans laquelle

le groupement $R_1$' représente un atome d'hydrogène ou un groupe alkyle à chaine linéaire ou ramifiée contenant jusqu'à 6 atomes de carbone ;
le groupement $R_2$' représente une simple liaison ou un groupe sélectionné parmi : - $(C_1-C_6)$alkyle-, - $(C_2-C_6)$alkényle-, -COO-, ou - $(C_1-C_6)$alkyle-COO-, de préférence -COO- ou $-(C_1-C_6)$alkyle-COO-;
le groupement Q représente -Z-L- où

le groupement Z représente une simple liaison ou

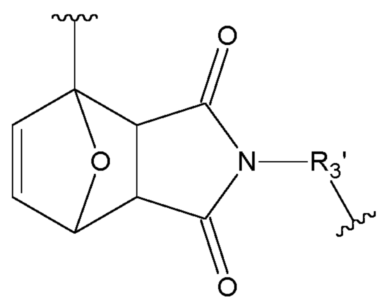

le groupement L représente une simple liaison ou un groupe sélectionné parmi : - $(C_1-C_6)$alkyle-, -$(C_2-C_6)$alkényle-, -$(C_2-C_6)$alkynyle-, -$(CONHR_4NHCOOR_5O)_m$-, -$(O\text{-}CO\text{-}NR_6)_m$-, -$(ORCHOHR_5)_m$-, -$(CH_2CHOHCH_2OR_4O)_m$-, -$(OR_4)_m$-, -$((CHOR_6)CH_2O\text{-}R4)_m$-, -$(R_4\text{-}COO\text{-}R_5)_m$-, - $NR_6$-, -$SO_2$-, -$SO_2NR_6$-, -O-, -S-, -$CONR_6$-, -aryle-, -$(\text{aryle-}R_4)_m$-, -$(\text{hétéroaryle-}R_4)_m$-, -$((C_3\text{-}C_8)\text{hétérocyclyle-}R_4)_m$-, -$((C_3\text{-}C_{14})\text{cycloalkyle-}R_4)_m$-, - $(C_1-C_6)$alkyle-$R_7$-$(C_1-C_6)$alkyle-, -$(C_2-C_6)$alkényle-$R_7$-$(C_1-C_6)$alkyle-, - $(C_1-C_6)$alkyle-$R_7$-$(C_2-C_6)$alkényle-,
ou - $(C_2-C_6)$alkényle-$R_7$-$(C_2-C_6)$alkényle-,

où les groupes $R_4$ et $R_5$ représente un groupe sélectionné parmi : simple liaison, -$(C_1-C_6)$alkyle-, -$(C_2-C_6)$alkényle-, -$NR_6$-, -$SO_2$-, -$SO_2NR_6$-, -O-, -S-, -$CONR_4$-, -aryle-, -$(\text{aryle-}R_4)_m$-, -$(\text{hétéroaryle-}R_4)_m$-, -$((C_3\text{-}C_8)$ hétérocyclyle) -$R_4)_m$-, - $((C_3\text{-}C_{14})\text{cycloalkyle-}R_4)_m$-aryle-, -hétéroaryle-, -$(C_3-C_8)$hétérocyclyle-, -$(C_3-C_{14})$cycloalkyle-, -$(C_1-C_6)$alkyle-OCO-, ou -$CH_2$-$(CHOR_3)CH_2O$-$(C_1-C_6)$alkyle- ;
où $R_6$ représente un groupe sélectionné parmi : atome d'hydrogène, -(Ci-Ce) alkyle, -$(C_2-C_6)$alkényle, -$NH_2$, -COOH, -$SO_2H$, -OH, -SH, -aryle, -hétéroaryle, -$(C_3-C_8)$hétérocyclyle ou -$(C_3-C_{14})$cycloalkyle, ;
où $R_7$ représente un groupe sélectionné parmi : simple liaison, -$(CONHR_4NHCOOR_5O)_m$ -, -$(O\text{-}CO\text{-}NR_4)_m$-, -$(COR_4)_m$-, - $(CH_2CHOHCH_2OR_4O)_m$-, -$(OR_4)_m$-, -$((CHOR_4)CH_2O\text{-}R_5)_m$-, -$(R_4\text{-}COO\text{-}R_5)_m$-, -$NR_6$-, -$SO_2$-, -$SO_2NR_4$-, -O-, -S-, -$CONR_4$-, -aryle-, - $(\text{aryle-}R_4)_m$-, -$(\text{hétéroaryle-}R_4)_m$-, -$((C_3\text{-}C_8)\text{hétérocyclyle)-}R_4)_m$-, -$((C_3\text{-}C_{14})\text{cycloalkyle-}R_4)_m$-, - $(C_1-C_6)$alkyle-OCO-, ou -$CH_2$-$(CHOR_3)CH_2O$-$(C_1-C_6)$alkyle- ;

avec n, représentant le nombre d'unités de répétition, compris entre 1 et 10,
avec m, représentant le nombre d'unités de répétition, compris entre 1 et 20, par exemple entre 1 et 10.

Ce procédé de synthèse d'un composé réticulant selon l'invention permet d'obtenir une grande diversité de composés réticulant de façon simple et rapide. En outre, lorsque le procédé de synthèse comporte une étape de purification permettant de supprimer les résidus des composés (II), (III), (IV), (V) alors il permet de meilleures performances notamment lors d'une utilisation dans des compositions pour polymère thermoplastique.

De plus, il a également été découvert qu'une pièce composite obtenue par le procédé de fabrication présente une teneur en monomère résiduel significativement réduite, en raison d'une meilleure conversion du monomère. La teneur en composé réticulant monomérique résiduel est inférieure à 5 % par rapport à la quantité du monomère utilisé.

Ainsi, l'invention porte en outre sur une composition comprenant au moins 80 % de composé réticulant de formule (I), de préférence au moins 90 %, de façon plus préférée au moins 95 % et de façon encore plus préférée au moins 99 %.

Si le procédé de synthèse est suivi d'une étape de purification et considérant des ratios de réactif optimisés pour assurer une conversion supérieure à 90 %, comme cela est présenté dans la présente demande, alors cette composition présente l'avantage de contenir moins de 5 % de fonctions diènes conjugués et diènophiles (i.e. aptes à subir une réaction d'addition de Diels-Alder) n'ayant pas fait l'objet d'une réaction d'addition de Diels-Alder (i.e. libres).

Cette composition peut, de façon particulièrement avantageuse, être utilisée dans une composition liquide destinée à la formation de polymère thermoplastique. Ainsi, la réticulation sera efficace et le polymère obtenu ne comportera pas ou peu de sous-produits de type diènes conjugués et diènophiles libres.

Cette composition liquide selon l'invention, de préférence de viscosité contrôlée, peut être utilisée dans de nombreux procédés industriels. Elle peut subir, après ajout d'au moins un armorceur radicalaire, une polymérisation rapide sans nécessiter une augmentation trop forte de la température.

Grâce à ce composé réticulant, la création d'un réseau de polymère n'est pas régie par la cinétique d'addition Diels-Alder mais uniquement pilotée par les paramètres de polymérisation spécifiques des deux fonctions vinyliques polymérisables en présence.

Cette composition nécessite en outre une faible concentration en composé réticulant et de basses concentrations sont avantageuses de façon à disposer d'un polymère thermoplastique pouvant à haute température être entièrement dé-réticulé et fluidifié. Cela permet une manipulation industrielle accélérée et facilitée.

En outre, la quantité de composés libres diènes conjugués/diénophile, aptes à former un produit d'addition Diels-Alder, dans la composition liquide selon l'invention est inférieure à 5 phr, de préférence inférieure à 1 phr, de façon plus préférée inférieure à 0,1 phr par rapport à la somme du monomère (méth)acrylique (M1) et du polymère (méth)acrylique (P1). Ainsi, pour un polymère formé à partir de cette composition liquide, cela réduit les risques de réactivité ultérieure ou de relargage.

L'invention porte en outre sur un procédé de préparation d'une composition liquide selon l'invention, ledit procédé comportant une première étape de mélange d'un composé réticulant, à un sirop comprenant un monomère (méth)acrylique (M1) ou un mélange de monomères (méth)acryliques, et/ou au moins un polymère (méth)acrylique (P1). De façon avantageuse, cette première étape correspond à la solubilisation du composé réticulant dans un sirop comprenant un monomère (méth)acrylique (M1) ou un mélange de monomères (méth)acryliques. En effet, comme cela sera détaillé après, un tel mélange permet une meilleure solubilisation du composé réticulant dans la composition et une meilleure réversibilité de la réaction de rétro Diels-Alder. Dans ce cas, le procédé comporte une seconde étape d'ajout d'au moins un polymère (méth)acrylique (P1) au mélange préparé dans la première étape.

Ce procédé de préparation peut inclure en outre l'ajout d'au moins un amorceur radicalaire. Cet ajout est de préférence réalisé peu de temps avant une étape de polymérisation.

L'invention porte en outre sur l'utilisation de la composition liquide selon l'invention pour fabriquer des formulations pour les arts graphiques tels que des encres ou des vernis, pour des revêtements, pour des adhésifs tels que des adhésifs structuraux, pour des peintures telles que des peintures routières, pour l'étanchéité de toits ou de sols, pour des enduits gélifiés (« gelcoats » en anglais) ou des couches de finition (« topcoats » en anglais) tels que pour les marbres artificiels, pour des chevilles chimiques ou des renforts pour ciment.

L'invention porte également sur l'utilisation de la composition liquide selon l'invention pour fabriquer des pièces thermoplastiques ou fabriquer des pièces composite ainsi que sur les procédés de fabrication associés.

Ces procédés de fabrication sont très rapides contrairement aux procédés actuels s'appuyant sur la réticulation par réaction d'addition Diels-Alder classique. En outre ils peuvent être conduits à des températures modérées c'est-à-dire par exemple inférieures à 200 °C.

L'invention porte en outre sur un matériau composite polymère comprenant une matrice (méth)acrylique thermoplastique et un substrat fibreux utilisé en tant que renfort, dans lequel le substrat fibreux est constitué de fibres longues, ledit matériau composite étant caractérisé en ce que la matrice (méth)acrylique thermoplastique est obtenue après polymérisation de la composition liquide, ledit substrat fibreux étant pré-imprégné avec la composition liquide selon l'invention. L'invention porte alors également sur une pièce mécanique ou élément structural constitué(e) du matériau composite selon l'invention.

Comme cela a été évoqué, ces matériaux composites polymères peuvent présenter l'avantage de comporter peu ou pas de résidus. Ainsi cela réduit les risques de réactivité ultérieure ou de relargage.

D'autres avantages et caractéristiques de l'invention apparaitront à la lecture de la description suivante donnée à titre d'exemple illustratif et non limitatif, en référence aux Figures annexées qui représentent :

- Figure 1, la cinétique de réaction Diels-Alder à 60°C suivi par RMN $^1$H entre FMA et BMI, lorsque le ratio FMA:BMI est de 2:1 (triangles) ou 4:1 (carrés).

- Figure 2, l'évolution du taux de conversion en fonction du temps pour les compositions contenant 5phr de composé réticulant selon l'invention avec une mise en suspension homogène (carré) ou une solubilisation (triangle).

- Figure 3, l'évolution du taux de conversion en fonction du temps et à différentes températures lors de la réaction de rétro Diels-Alder dans du DMF deutéré telle que suivi par RMN $^1$H.

[**Description** de l'invention]

L'abréviation « phr » désigne des parties en poids par cent parties de composition. Par exemple 1 phr d'amorceur radicalaire dans la composition signifie que 1 kg d'amorceur radicalaire est ajouté à 100 kg de composition.

L'abréviation « ppm » désigne des parties en poids par million de parties de composition. Par exemple, 1000 ppm d'un composé dans la composition signifie que 0,1 kg de composé est présent dans 100 kg de composition.

L'expression « composite polymère », au sens de l'invention, désigne un matériau multicomposant comprenant au moins deux composants non miscibles dans lequel au moins un composant est un polymère et l'autre composant peut par exemple être un renfort fibreux.

On entend par « renfort fibreux » ou « substrat fibreux », au sens de l'invention, plusieurs fibres, des stratifils unidirectionnels ou un mat à filament continu, des tissus, des feutres ou des non-tissés qui peuvent être sous la forme

de bandes, nappes, tresses, mèches ou pièces.

On entend par « matrice », une matière servant de liant et capable de transférer des efforts au renfort fibreux. La « matrice de polymère » comporte des polymères mais peut également comporter d'autres composés ou matériaux. Ainsi, la « matrice de polymère (méth)acrylique », se rapporte à tout type de composés, polymères, oligomères, copolymères ou copolymères à blocs, acryliques et méthacryliques. Cependant, on ne sortirait pas du cadre de l'invention si la matrice de polymère (méth)acrylique comprend jusqu'à 20% en poids, de préférence moins de 10% en poids, de façon plus préférée moins de 5% en poids d'autres monomères non acryliques, choisis par exemple dans le groupe : butadiène, isoprène, styrène, styrène substitué tel que l'$\alpha$-methylstyrène ou le tert-butylstyrène, cyclosiloxanes, vinylnaphthalènes et vinyl pyridines.

Par « polymère », on entend soit un copolymère soit un homopolymère. On entend par « copolymère », un polymère regroupant plusieurs unités monomères différentes et par « homopolymère », un polymère regroupant des unités monomères identiques. On entend par « copolymère à blocs », un polymère comprenant une ou plusieurs séquences ininterrompues de chacune des espèces polymères distinctes, les séquences polymères étant chimiquement différentes l'une de l'autre et étant liées entre elles par une liaison covalente. Ces séquences polymères sont encore dénommées blocs polymères.

Le terme « amorceur radicalaire », au sens de l'invention, désigne un composé qui peut démarrer/initier la polymérisation d'un monomère ou de monomères.

Le terme « polymérisation », au sens de l'invention, désigne le procédé de conversion d'un monomère ou d'un mélange de monomères en un polymère.

Le terme « monomère », au sens de l'invention, désigne une molécule qui peut subir une polymérisation.

On entend par « polymère thermoplastique », au sens de l'invention, un polymère généralement solide à température ambiante, pouvant être cristallin, semi-cristallin ou amorphe, et qui se ramollit lors d'une augmentation de température, en particulier après passage de sa température de transition vitreuse (Tg) et s'écoule à plus haute température et pouvant observer une fusion franche au passage de sa température dite de fusion (Tf) (lorsqu'il est semi-cristallin), et qui redevient solide lors d'une diminution de température en dessous de sa température de fusion et en dessous de sa température de transition vitreuse. Cela s'applique également pour des polymères thermoplastiques légèrement réticulés par la présence de monomères ou oligomères multifonctionnels dans la formulation du « sirop » (méth)acrylate, en pourcentage massique de préférence moins de 10%, de préférence moins de 5% et de façon préférée moins de 2% qui peuvent être thermoformés lorsqu'ils sont chauffés au-dessus de la température de ramollissement.

On entend par « polymère thermodurcissable », au sens de l'invention, une matière plastique qui se transforme de manière irréversible par polymérisation en un réseau polymère insoluble.

On entend par « monomère (méth)acrylique », tout type de monomère acrylique et méthacrylique.

On entend par « polymère (méth)acrylique », un polymère comprenant essentiellement des monomères (méth)acryliques qui représentent au moins 50% en poids ou plus du polymère (méth)acrylique.

Le terme « PMMA », au sens de l'invention, désigne des homo- et copolymères de méthacrylate de méthyle (MMA), le rapport en poids de MMA dans le PMMA étant de préférence d'au moins 70 % en poids pour le copolymère de MMA.

L'expression « groupe alkyle, linéaire ou ramifiée, contenant jusqu'à 6 atomes de carbone » tel qu'utilisée dans la présente invention (aussi appelée alkyle en $C_1$-$C_6$), correspond à une chaîne hydrocarbonée saturée, linéaire ou ramifiée, contenant de 1 à 6 atomes de carbone ou à une chaîne hydrocarbonée insaturée, linéaire ou ramifiée, contenant de 2 à 6 atomes de carbone. Une chaîne hydrocarbonée saturée, linéaire ou ramifiée, contenant de 1 à 6 atomes de carbone comprend, sans s'y limiter, les groupes méthyle, éthyle, n-propyle, isopropyle, n-butyle, isobutyle, sec-butyle, t-butyle, n-pentyle et similaires. Une chaîne hydrocarbonée insaturée, linéaire ou ramifiée, contenant de 2 à 6 atomes de carbone comprend au moins une double ou une triple liaison, et inclut, sans s'y limiter, les groupes éthène, propène, butène, pentène, éthényle, propényle, butényle, pentényle et similaires.

Le terme « groupe aryle », tel qu'utilisé dans la présente invention, désigne un groupe hydrocarboné aromatique comprenant de préférence 6 à 10 atomes de carbone et comprenant un ou plusieurs, notamment 1 ou 2, cycles condensés, comme par exemple un groupe phényle ou un groupe naphtyle. Avantageusement, cela désigne un groupe phényle.

Le terme « -(alkyle en $C_1$-C6)-aryle », tel qu'utilisé dans la présente invention, désigne un groupe aryle tel que défini ci-dessus lié à la molécule via un groupe alkyle en C1 à C6 tel que défini ci-dessus. En particulier, le groupe -(alkyle en $C_1$-$C_6$)-aryle selon l'invention est un groupe benzyle. Pour les groupes comprenant deux sous-groupes ou plus, l'attachement est indiqué par « - ». Par exemple, « -(alkyle en $C_1$-$C_5$)-aryle » désigne un radical alkyle lié à un radical aryle dans lequel l'alkyle est lié au reste de la molécule. Pour les groupes comprenant un attachement de chaque côté, par exemple, « -(alkyle en $C_1$-$C_5$)-aryle- », cela désigne un radical alkyle lié à un radical aryle dans lequel l'alkyle ou l'aryle sont liés au reste de la molécule et cela englobe aussi bien un groupement -(alkyle en $C_1$-$C_5$)-aryle- qu'un groupement -aryle-(alkyle en $C_1$-$C_5$)-.

Les groupements selon l'invention, par exemple le groupe aryle ou le groupe cycloalkyle, peuvent être éventuellement substitués selon la présente invention avec un ou plusieurs groupes choisis indépendamment dans le groupe consistant en alkyle, alkoxyle (alcoxyle), hydroxyle, carboxyle ou ester. Des exemples de groupes phényle éventuellement substitués sont le méthoxyphényle, le diméthoxyphényle et le carboxyphényle. Alternativement, ils ne sont substitués que si cela est explicitement spécifié. Le terme « éventuellement substitué » tel qu'utilisé ici signifie que l'un quelconque des atomes d'hydrogène peut être remplacé par un substituant, tel qu'un groupement carboxyle.

**Le composé réticulant** désigne un composé réticulant de formule (I)

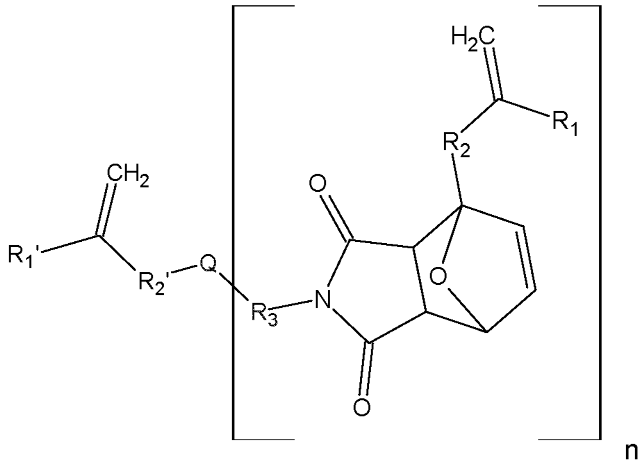

(I)

dans laquelle :

le groupement Q représente -Z-L- où

le groupement Z représente une simple liaison ou

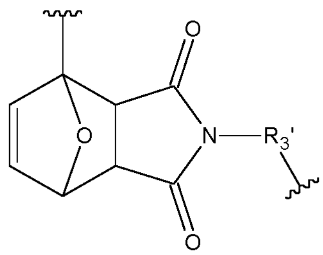

,

le groupement L représente une simple liaison ou un groupe sélectionné parmi : - $(C_1-C_6)$alkyle-, -$(C_2-C_6)$alkényle-, -$(C_2-C_6)$alkynyle-, -$(CONHR_4NHCOOR_5O)_m$-, -$(O-CO-NR_6)_m$-, -$(ORCHOHR_5)_m$-, -$(CH_2CHOHCH_2OR_4O)_m$-, -$(OR_4)_m$-, -$((CHOR_6)CH_2O-R_4)_m$-, -$(R_4-COO-R_5)_m$-, - $NR_6$-, -$SO_2$-, -$SO_2NR_6$-, -O-, -S-, -$CONR_6$-, -aryle-, -(aryle-$R_4)_m$-, -(hétéroaryle-$R_4)_m$-, -($(C_3-C_8)$hétérocyclyle-$R_4)_m$-, -($(C_3-C_{14})$cycloalkyle-$R_4)_m$-, - $(C_1-C_6)$alkyle-$R_7$-$(C_1-C_6)$alkyle-, -$(C_2-C_6)$alkényle-$R_7$-$(C_1-C_6)$alkyle-, - $(C_1-C_6)$alkyle-$R_7$-$(C_2-C_6)$alkényle-,
ou - $(C_2-C_6)$alkényle-$R_7$-$(C_2-C_6)$alkényle-,

où les groupes $R_4$ et $R_5$ sont identiques ou différents et représentent un groupe sélectionné parmi : simple liaison, -$(C_1-C_6)$alkyle-, -$(C_2-C_6)$alkényle-, -$NR_6$-, -$SO_2$-, -$SO_2NR_6$-, -O-, -S-, -$CONR_6$-, -aryle-, -(aryle-$R_6)_m$-, - (hétéroaryle-$R_6)_m$-, - ($(C_3-C_8)$hétérocyclyle)-$R_6)_m$-, -($(C_3-C_{14})$cycloalkyle-$R_6)_m$-aryle-, -hétéroaryle-, -$(C_3-C_8)$ hétérocyclyle-, -$(C_3-C_{14})$cycloalkyle-, -$(C_1-C_6)$alkyle-OCO-, ou -$CH_2$-$(CHOR_3)CH_2O$-$(C_1-C_6)$alkyle- ;

où $R_6$ représente un groupe sélectionné parmi : atome d'hydrogène, -(Ci-Ce) alkyle, -$(C_2-C_6)$alkényle, -$NH_2$, -COOH, -$SO_2H$, -OH, -SH, -aryle, -hétéroaryle, -$(C_3-C_8)$hétérocyclyle, ou -$(C_3-C_{14})$cycloalkyle ;

où $R_7$ représente un groupe sélectionné parmi : simple liaison, -$(CONHR_4NHCOOR_5O)_m$-, -$(O-CO-NR_4)_m$-, -$(COR_4)_m$-, -$(OR_4)_m$-, -$(CH_2CHOHCH_2OR_4O)_m$-, -$((CHOR_4)CH_2O-R_5)_m$-, -$(R_4-COO-R_5)_m$-, -$NR_6$-, -$SO_2$-, -$SO_2NR_6$-, -O-, -S-, -$CONR_6$-, -aryle-, -(aryle-$R_4)_m$-, - (hétéroaryle-$R_4)_m$-, - ($(C_3-C_8)$hétérocyclyle) -$R_4)_m$-,

- $((C_3\text{-}C_{14})$cycloalkyle-$R_4)_m$-, - $(C_1\text{-}C_6)$alkyle-OCO-, ou -$CH_2$-($CHOR_3$)$CH_2O$-$(C_1\text{-}C_6)$alkyle- ;

les groupements $R_1$ et $R_1$' sont identiques ou différents et représentent un atome d'hydrogène ou un groupe alkyle à chaine linéaire, ou ramifiée contenant jusqu'à 6 atomes de carbone ;
les groupements $R_2$ et $R_2$' sont identiques ou différents et représentent une simple liaison ou un groupe sélectionné parmi : - $(C_1\text{-}C_6)$alkyle-, - $(C_2\text{-}C_6)$alkényle-, -COO-, ou -COO- $(C_1\text{-}C_6)$alkyle-, de préférence -COO- ou -COO-$(C_1\text{-}C_6)$alkyle- ;
les groupements $R_3$ et $R_3$' sont identiques ou différents et représentent un groupe sélectionné parmi : -NHCOO- ;

avec n, représentant le nombre d'unités de répétition, compris entre 1 et 10,
avec m, représentant le nombre d'unités de répétition, compris entre 1 et 20, par exemple entre 1 et 10.

Ainsi, le composé réticulant comporte au moins deux fonctions vinyliques polymérisables et au moins un groupement de type époxyisoindole, produit d'addition de Diels-Alder, apte à subir une réaction de rétro Diels-Alder.
De façon particulière, le composé réticulant peut comporter un seul groupement de type époxyisoindole, le groupement Z représente alors une simple liaison.
Alternativement, le composé réticulant peut comporter plusieurs groupements de type époxyisoindole. Dans ce cas, le groupement Z représente

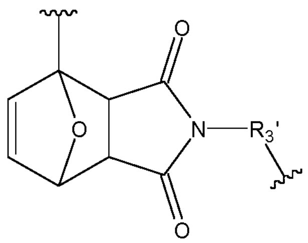

et n est compris entre 1 et 10, de préférence entre 1 et 5.
De façon préférée, les groupement vinyliques sont des groupement (meth)acrylates, très réactifs dans le cadre de réactions de polymérisation (les groupements $R_3$ et $R_3$' représentent -COO- ou - $(C_1\text{-}C_6)$alkyle-COO-) .
Ainsi, le composé réticulant est avantageusement choisi dans le groupe constitué des composés de formule (Ia)

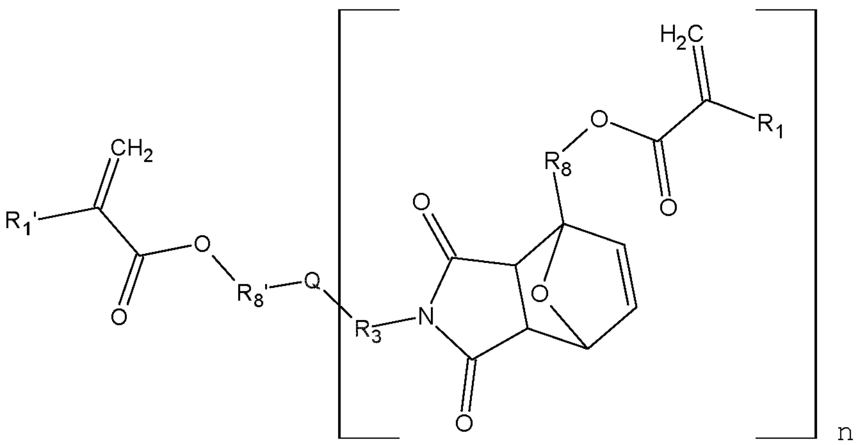

(Ia)

dans laquelle :
les groupements $R_8$ et $R_8$' sont identiques ou différents et représentent une simple liaison ou un groupe alkyle à chaine linéaire, ou ramifiée contenant jusqu'à 6 atomes de carbone.
De façon particulière, lorsque le composé réticulant ne comporte qu'un seul groupement de type époxyisoindole (Z est une simple liaison), le composé réticulant est choisi dans le groupe constitué des composés de formule (Ib)

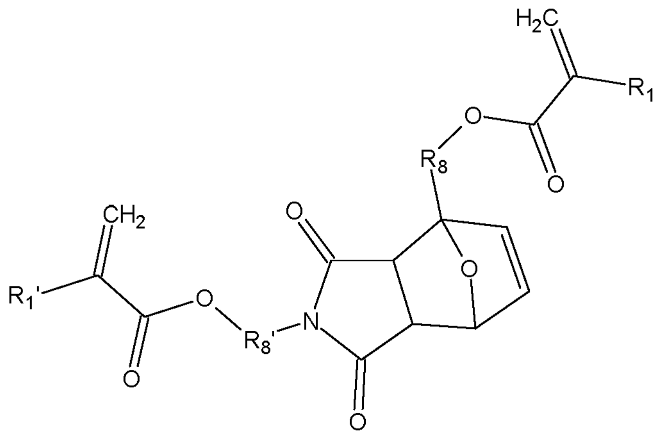

(Ib)

De façon particulière, lorsque le composé réticulant comporte au moins deux groupements de type époxyisoindole, le composé réticulant choisi dans le groupe constitué des composés de formule (Ic)

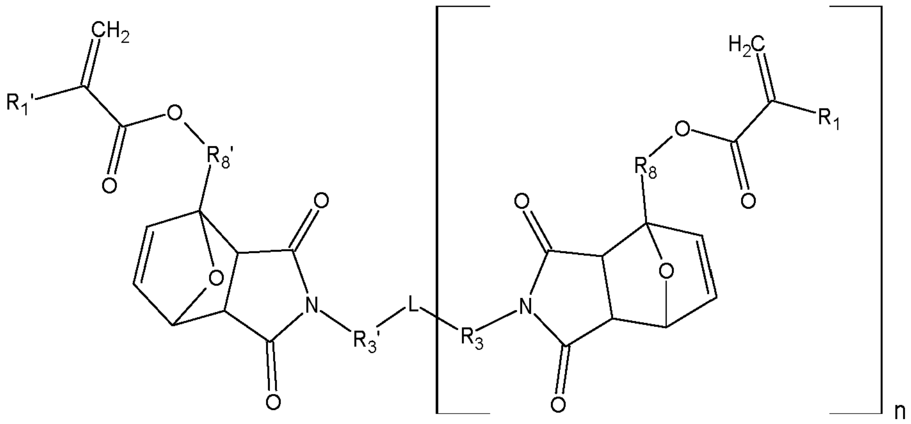

(Ic)

Les groupements de type époxyisoindole, produits d'addition de Diels-Alder, sont séparés par un espaceur « L ». De façon avantageuse, les produits d'addition de Diels-Alder sont liés à cet espaceur par une fonction uréthane générée lors d'une réaction isocyanate - hydroxyle. Ainsi, de façon préférée, le composé réticulant choisi dans le groupe constitué des composés de formule (Id)

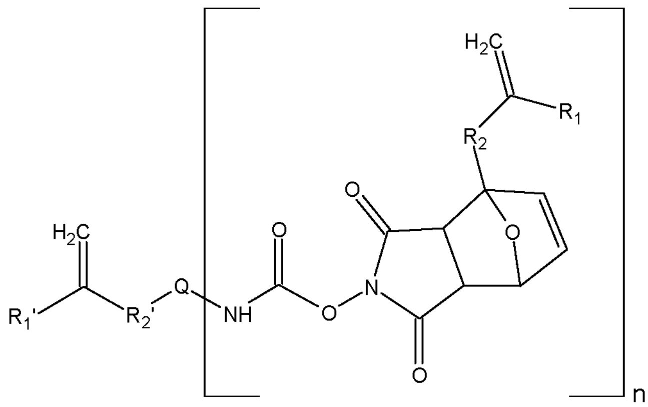

(Id)

De façon particulière, le composé réticulant ne comporte qu'un seul groupement époxyisoindole (Z est une simple liaison) et est choisi dans le groupe constitué des composés de formule (Ie)

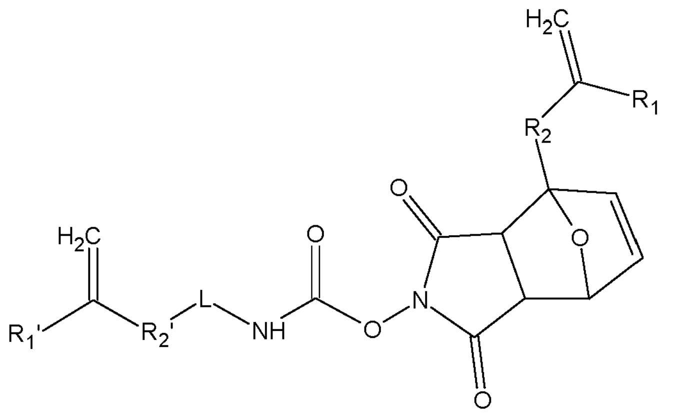

(Ie)

Alternativement, le composé réticulant comporte au moins deux fonctions époxyisoindole et est choisi dans le groupe constitué des composés de formule (If)

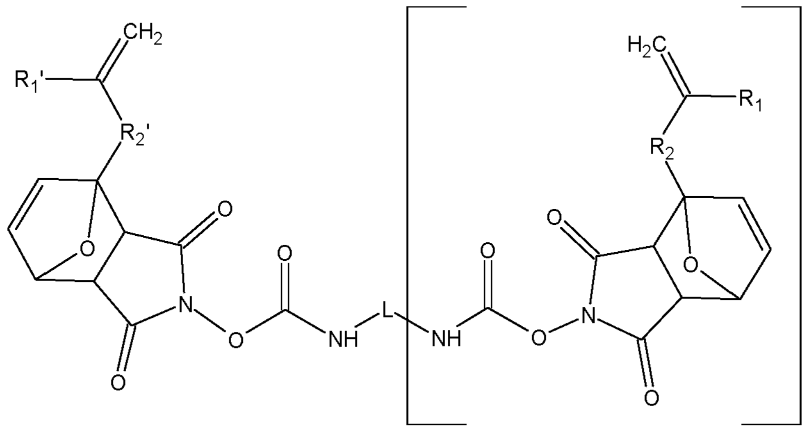

n (If)

De façon encore plus préférée, le composé réticulant selon l'invention comporte une fonction uréthane par exemple générée lors d'une réaction isocyanate - hydroxyle et des fonctions (méth)acrylate. Le composé réticulant peut alors être choisi dans le groupe constitué des composés de formule (Ig)

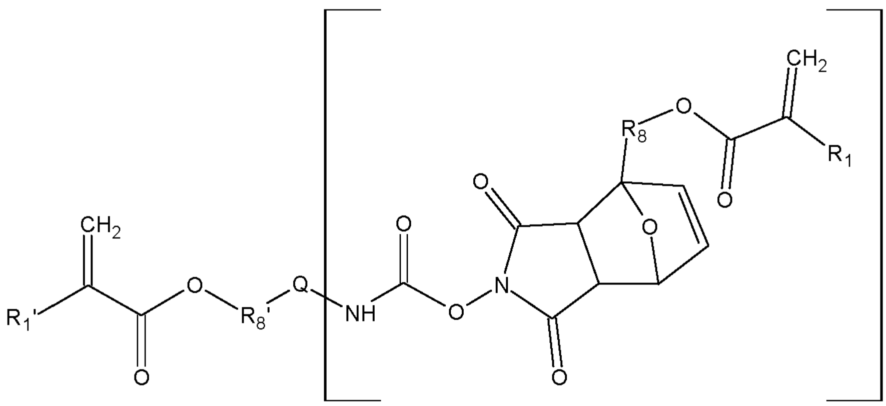

n (Ig)

De façon encore plus préférée, le composé réticulant selon l'invention comporte une fonction uréthane par exemple générée lors d'une réaction isocyanate - hydroxyle et des fonctions (méth)acrylique. Le composé réticulant peut alors être choisi dans le groupe constitué des composés de formule (Ig)

Alternativement, le composé réticulant comporte des fonctions (méth)acryliques et une seule fonction époxyisoindole et est choisi dans le groupe constitué des composés de formule (Ih)

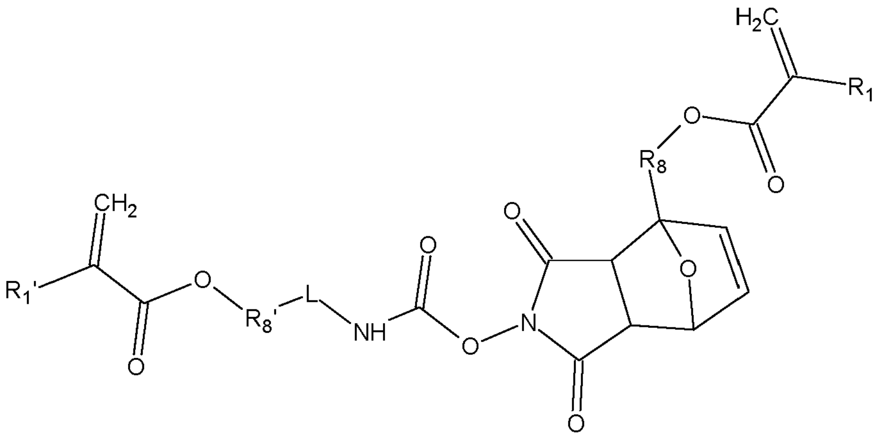

(Ih)

dans laquelle :
les groupements $R_8$ et $R_8$' sont identiques ou différents et représentent une simple liaison ou un groupe alkyle à chaine linéaire, ou ramifiée contenant jusqu'à 6 atomes de carbone.

Alternativement, le composé réticulant comporte des fonctions (méth)acryliques et au moins deux fonctions époxyisoindole et est choisi dans le groupe constitué des composés de formule (Ii)

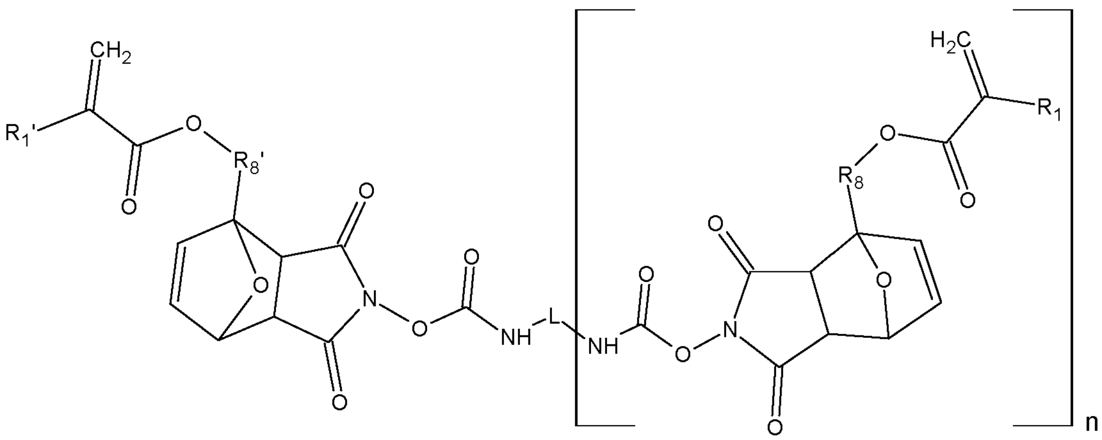

(Ii)

dans laquelle :
les groupements $R_8$ et $R_8$' sont identiques ou différents et représentent une simple liaison ou un groupe alkyle à chaine linéaire, ou ramifiée contenant jusqu'à 6 atomes de carbone.

Par exemple, le composé réticulant peut être choisi le composé de formule (B) ou de formule (C) tel que représenté ci-dessous :

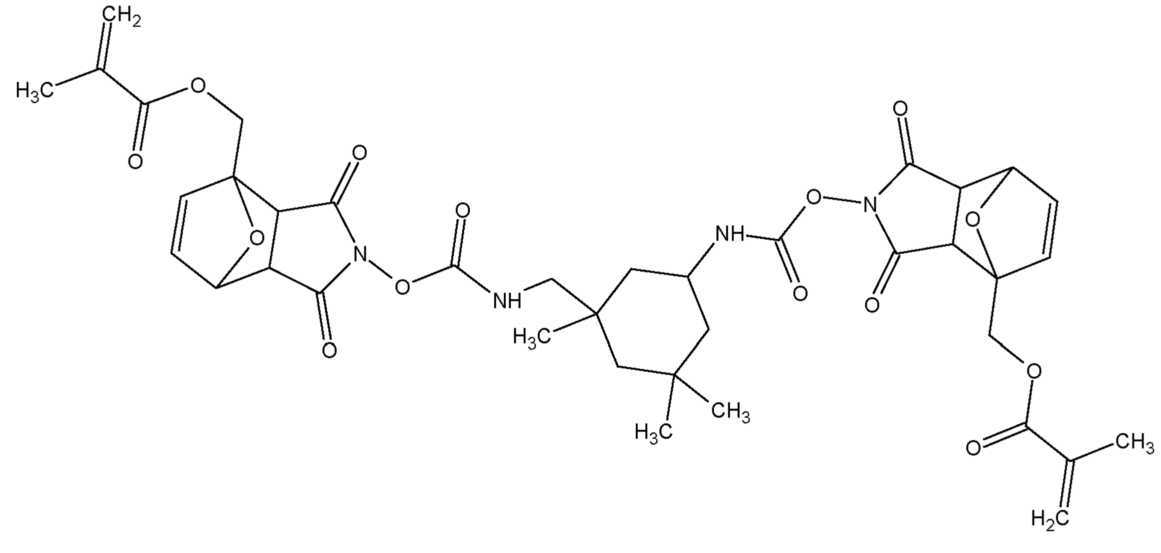

(B)

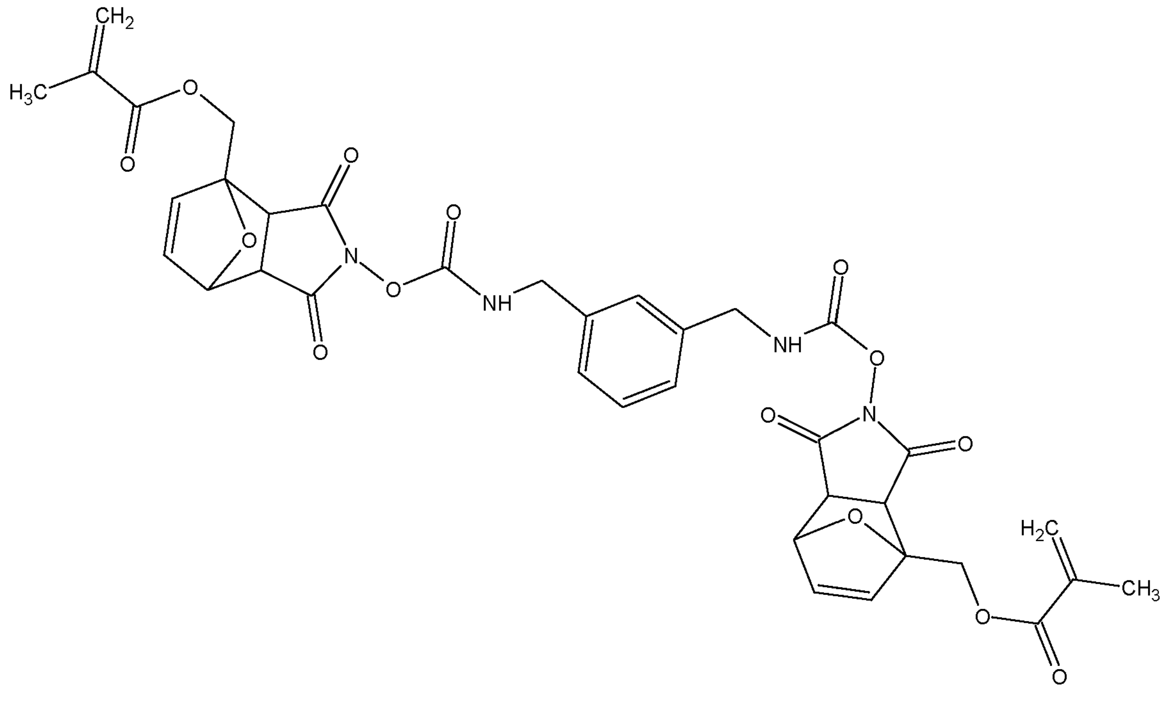

(C)

Le composé réticulant peut être utilisé en suspension homogène ou solubilisé dans une composition liquide et plus particulièrement dans une solution de monomère (méth)acrylique (M1) ou un mélange de monomères (méth)acryliques. En effet, les inventeurs ont montré qu'une solubilisation du composé réticulant permet d'obtenir une meilleure réversibilité de la réticulation d'un polymère thermoplastique comportant ledit composé réticulant. Ainsi, avantageusement, le composé réticulant selon l'invention présente une solubilité supérieure à 20 g/L, de préférence 40 g/L dans une solution de monomère (méth)acrylique (M1) ou un mélange de monomères (méth) acryliques à 25°C. Dans le cas de deux liquides, le composé réticulant selon l'invention, lorsque mélangé à une solution de monomère (méth)acrylique (M1) ou un mélange de monomères (méth)acryliques à 25°C, ne forme qu'une seule phase.

De façon particulière, le composé réticulant selon l'invention présente une température de rétro Diels-Alder supérieure à 150 °C, de préférence supérieure à 200 °C.

De façon particulière, le groupement L représente un groupe sélectionné parmi : - (Ci-Ce) alkyle-, -($C_2$-$C_6$)alkényle-, -($C_2$-$C_6$)alkynyle-, -($CONHR_4NHCOOR_5O)_m$-, -($O$-$CO$-$NR_6)_m$-, -($OR_4CHOHR_5)_m$-, - ($CH_2CHOHCH_2OR_4O)_m$-, -($OR_4)_m$-, -(($CHOR_6)CH_2O$-$R_4)_m$-, -($R_4$-$COO$-$R_5)_m$-, -$NR_6$-, -$SO_2$-, -$SO_2NR_6$-, -O-, -S-, -$CONR_6$-, -aryle-, -(aryle-$R_4)_m$-, - (hétéroaryle-$R_4)_m$-, -(($C_3$-$C_8$)hétérocyclyle-$R_4)_m$-, -(($C_3$-$C_{14}$)cycloalkyle-$R_4)_m$-, - ($C_1$-$C_6$)alkyle-$R_7$-($C_1$-$C_6$)alkyle-, -($C_2$-$C_6$)alkényle-$R_7$-($C_1$-$C_6$)alkyle-, -(Ci-Ce) alkyle-$R_7$-($C_2$-$C_6$)alkényle-, ou -($C_2$-$C_6$)alkényle-$R_7$-($C_2$-$C_6$)alkényle-.

De façon préférée, le groupement L représente un groupe sélectionné parmi : $-(C_1-C_6)$alkyle-, $-(C_2-C_6)$alkényle-, $-(CONHR_4NHCOOR_5O)_m-$, $-(O-CO-NR_6)_m-$, $-(OR_4CHOHR_5)_m-$, $-(CH_2CHOHCH_2OR_4O)_m-$, $-(OR_4)_m-$, $-((CHOR_6)CH_2O-R_4)_m-$, $-(R_4-COO-R_5)_m-$ -aryle-, -(aryle-$R_4$) $_m$-, - (hétéroaryle-$R_4$)$_m$-, -(($C_3$-$C_8$)hétérocyclyle-$R_4$)$_m$-, -(($C_3$-$C_{14}$)cycloalkyle-$R_4$)$_m$-, - ($C_1$-$C_6$)alkyle-$R_7$-($C_1$-$C_6$)alkyle-, - ($C_2$-$C_6$)alkényle-$R_7$-($C_1$-$C_6$)alkyle-, -(Ci-Ce) alkyle-$R_7$-($C_2$-$C_6$)alkényle-, ou -($C_2$-$C_6$)alkényle-$R_7$-($C_2$-$C_6$)alkényle-.

De façon plus préférée le groupement L présente une masse molaire d'au moins 200 g/mol, de préférence d'au moins 500 g/mol.

De façon particulière, les groupes $R_4$ et $R_5$ sont identiques ou différents et représentent un groupe sélectionné parmi : simple liaison, -($C_1$-$C_6$)alkyle-, -($C_2$-$C_6$)alkényle-, -$NR_6$-, -$SO_2$-, -$SO_2NR_6$-, -O-, -S-, -$CONR_6$-, -aryle-, -(aryle-$R_6$)$_m$-, -(hétéroaryle-$R_6$)$_m$-, -(($C_3$-$C_8$)hétérocyclyle)-$R_6$)$_m$-, -(($C_3$-$C_{14}$)cycloalkyle-$R_6$)$_m$-aryle-, - hétéroaryle-, -($C_3$-$C_8$)hétérocyclyle-, -($C_3$-$C_{14}$)cycloalkyle-, -($C_1$-$C_6$)alkyle-OCO-, ou -$CH_2$-($CHOR_3$)$CH_2O$-($C_1$-$C_6$)alkyle-

De façon particulière, le groupe $R_6$ peut représenter un groupe sélectionné parmi : atome d'hydrogène, -($C_1$-$C_6$)alkyle, -($C_2$-$C_6$)alkényle, -$NH_2$, -COOH, -$SO_2H$, -OH, -SH, -aryle, -hétéroaryle, -($C_3$-$C_8$)hétérocyclyle, ou -($C_3$-$C_{14}$)cycloalkyle ;

De façon particulière, le groupe $R_7$ peut représenter un groupe sélectionné parmi : simple liaison, $-(CONHR_4NHCOOR_5O)_m-$, $-(O-CO-NR_4)_m-$, $-(COR_4)_m-$, $-(OR_4)_m-$, $-(CH_2CHOHCH_2OR_4O)_m-$, $-((CHOR_4)CH_2O-R_5)_m-$, $-(R_4-COO-R_5)_m-$, -$NR_6$-, -$SO_2$-, - $SO_2NR_6$-, -O-, -S-, -$CONR_6$-, -aryle-, -(aryle-$R_4$)$_m$-, -(hétéroaryle-$R_4$)$_m$-, -(($C_3$-$C_8$) hétérocyclyle)-$R_4$)$_m$-, -(($C_3$-$C_{14}$)cycloalkyle-$R_4$)$_m$-, -($C_1$-$C_6$)alkyle-OCO-, ou -$CH_2$-($CHOR_3$)$CH_2O$-($C_1$-$C_6$)alkyle- ;

Les groupements R1 et R1' sont identiques ou différents et représentent un atome d'hydrogène ou un groupe alkyle à chaine linéaire, ou ramifiée contenant jusqu'à 6 atomes de carbone. De préférence, les groupements R1 et R1' sont identiques ou différents et représentent un atome d'hydrogène ou un groupe alkyle à chaine linéaire contenant jusqu'à 6 atomes de carbone. De façon plus préférée, les groupements R1 et R1' sont identiques ou différents et représentent un atome d'hydrogène ou un méthyle.

Les groupements $R_2$ et $R_2$' sont identiques ou différents et représentent une simple liaison ou un groupe sélectionné parmi : - ($C_1$-$C_6$)alkyle-, - ($C_2$-$C_6$)alkényle-, -COO-, - ($C_1$-$C_6$)alkyle-COO-. De préférence, les groupements $R_2$ et $R_2$' sont identiques ou différents et représentent un groupe -COO- ou -($C_1$-$C_6$)alkyle-COO-. De façon plus préférée, les groupements $R_2$ et $R_2$' sont identiques ou différents et représentent un groupe -($C_1$-$C_6$)alkyle-COO-. De façon encore plus préférée, les groupements $R_2$ et $R_{2'}$ représentent un groupe -$CH_2$-COO-.

Les groupements $R_3$ et $R_3$' sont identiques ou différents et représentent un groupe sélectionné parmi : -NHCOO-.

De façon encore plus préférée, les groupements $R_3$ et $R_3$' représentent un groupe -NHCOO-.

Le **procédé de synthèse du composé réticulant** comprend une étape de réaction d'addition Diels-Alder entre un maléimide de formule (II)

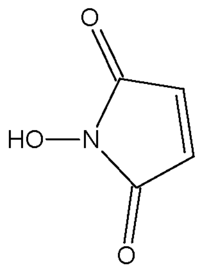

(II)

et une molécule de formule (III)

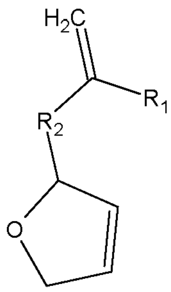

(III)

dans laquelle :

le groupement $R_1$ représente un atome d'hydrogène ou un groupe alkyle à chaîne linéaire, ou ramifiée contenant jusqu'à 6 atomes de carbone ;

le groupement $R_2$ représente une simple liaison ou un groupe sélectionné parmi : -($C_1$-$C_6$)alkyle-, -($C_2$-$C_6$)alkényle-, -COO-, ou -($C_1$-$C_6$)alkyle-COO-, de préférence -COO- ou -($C_1$-$C_6$)alkyle-COO- ;

de façon à former un produit d'addition Diels-Alder ;

et une étape de réaction entre ledit produit d'addition Diels-Alder avec une molécule comportant au moins une fonction isocyanate sélectionnée parmi les molécules de formule (IV) ou de formule (V)

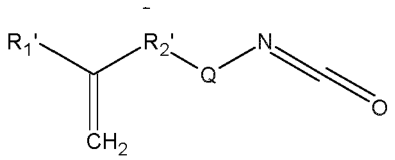
(IV)

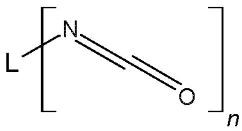
(V)

dans laquelle
le groupement Q représente -Z-L- où

le groupement Z représente une simple liaison ou

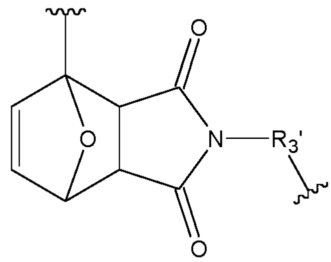
,

le groupement L représente une simple liaison ou un groupe sélectionné parmi : - ($C_1$-$C_6$)alkyle-, - ($C_2$-$C_6$)alkényle-, -($C_2$-$C_6$)alkynyle-, - ($CONHR_4NHCOOR_5O$)$_m$-, - (O-CO-$NR_6$)$_m$-, - ($ORCHOHR_5$)$_m$-, - ($CH_2CHOHCH_2OR_4O$)$_m$-, - ($OR_4$)$_m$-, - (($CHOR_6$)$CH_2O$-R4)$_m$-, - ($R_4$-COO-$R_5$)$_m$-, - $NR_6$-, -$SO_2$-, -$SO_2NR_6$-, -O-, -S-, -$CONR_6$-, -aryle-, -(aryle-$R_4$)$_m$-, -(hétéroaryle-$R_4$)$_m$-, -(($C_3$-$C_8$) hétérocyclyle-$R_4$)$_m$-, -(($C_3$-$C_{14}$)cycloalkyle-$R_4$)$_m$-, - ($C_1$-$C_6$)alkyle-$R_7$-($C_1$-$C_6$)alkyle-, -($C_2$-$C_6$)alkényle-$R_7$-($C_1$-$C_6$)alkyle-, - ($C_1$-$C_6$)alkyle-$R_7$-($C_2$-$C_6$)alkényle-, - ($C_2$-$C_6$)alkényle-$R_7$-($C_2$-$C_6$)alkényle-,

où les groupes $R_4$ et $R_5$ représente un groupe sélectionné parmi : simple liaison, -($C_1$-$C_6$)alkyle-, -($C_2$-$C_6$)alkényle-, -$NR_6$-, -$SO_2$-, -$SO_2NR_6$-, -O-, -S-, -$CONR_4$-, -aryle-, -(aryle-$R_4$)$_m$-, -(hétéroaryle-$R_4$)$_m$-, -(($C_3$-$C_8$) hétérocyclyle) -$R_4$)$_m$-, - (($C_3$-$C_{14}$)cycloalkyle-$R_4$)$_m$-aryle-, -hétéroaryle-, -($C_3$-$C_8$)hétérocyclyle-, -($C_3$-$C_{14}$)cycloalkyle-, -($C_1$-$C_6$)alkyle-OCO-, ou -$CH_2$-($CHOR_3$)$CH_2O$-($C_1$-$C_6$)alkyle- ;
où $R_6$ représente un groupe sélectionné parmi : atome d'hydrogène, -($C_1$-$C_6$)alkyle-, -($C_2$-$C_6$)alkényle-, -$NH_2$, - COOH, -$SO_2$H, -OH, -SH, -aryle, -hétéroaryle, -($C_3$-$C_8$)hétérocyclyle ou -($C_3$-$C_{14}$)cycloalkyle ;
où $R_7$ représente un groupe sélectionné parmi : simple liaison, -($CONHR_4NHCOOR_5O$)$_m$-, -(O-CO-$NR_4$)$_m$-, -($COR_4$)$_m$-, - ($CH_2CHOHCH_2OR_4O$)$_m$-, -($OR_4$)$_m$-, -(($CHOR_4$)$CH_2O$-$R_5$)$_m$-, -($R_4$-COO-$R_5$)$_m$-, -$NR_6$-, -$SO_2$-, -$SO_2NR_4$-, -O-, -S-, -$CONR_4$-, -aryle-, - (aryle-$R_4$)$_m$-, -(hétéroaryle-$R_4$)$_m$-, -(($C_3$-$C_8$) hétérocyclyle)-$R_4$)$_m$-, -(($C_3$-$C_{14}$)cycloalkyle-$R_4$)$_m$-, - ($C_1$-$C_6$)alkyle-OCO-, ou - $CH_2$-($CHOR_3$)$CH_2O$-($C_1$-$C_6$)alkyle- ;

Ce procédé de synthèse permet avantageusement d'obtenir un composé réticulant de formule (Ie) ou de formule (Ii)

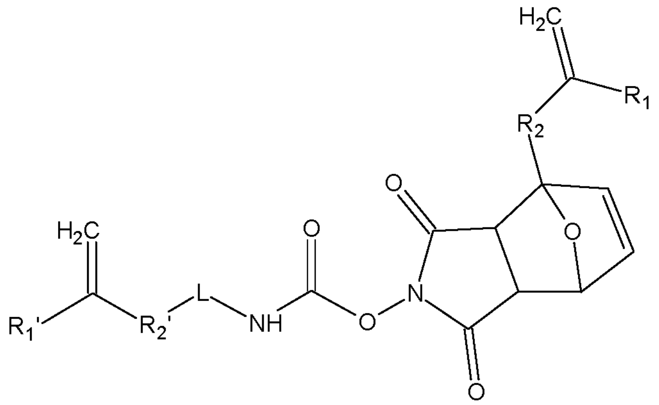

(Ie)

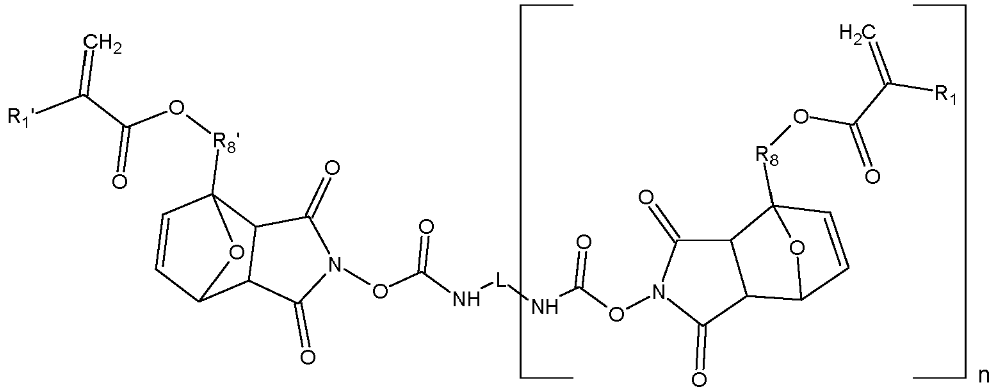

(Ii)

Dans un premier temps, il y a la synthèse du groupement réversible par une réaction d'addition Diels-Alder entre un maléimide portant une fonction hydroxyle (composé de formule (II)) et un furfuryl portant une fonction réactive composé de formule (III). La réaction d'addition Diels-Alder entre un maléimide de formule (II) et le furfuryl de formule (III) peut par exemple être réalisée à 60°C en présence de tétrahydrofurane (THF).

Une fois le produit Diels-Alder obtenu, il devient aisé de faire réagir, en présence d'un catalyseur, les fonctions hydroxyles sur une fonction isocyanate d'une molécule dont l'architecture, et notamment le groupement L, aura été préalablement sélectionné.

L'utilisation d'une molécule comprenant au moins un isocyanate de formule (IV) ou (V) permet d'atteindre des rendements élevés et se basant sur la chimie des uréthanes.

Avantageusement, le ratio molaire entre les composés de formule (III) et les composés de formule (II) est supérieur à 2, de préférence supérieur ou égale à 3 et de façon encore plus préférée supérieur ou égale à 4. En effet, il a été montré qu'un excès de Furfuryl méthacrylate conduit à un rendement de réaction plus élevé (≈ 98%).

De façon à obtenir un taux de conversion maximal, la réaction entre les composés de formule (III) et les composés de formule (II) peut présenter une durée d'au moins 5 heures, de préférence d'au moins 10 heures, de façon plus préférée d'au moins 20 heures et de façon encore plus préférée d'au moins 40 heures.

Le procédé de synthèse comporte en outre une étape ultérieure de purification visant à supprimer les molécules de formule (II), (III), (IV) n'ayant pas réagi pour former le composé réticulant. Les procédés de purification peuvent par exemple être sélectionnés par la chromatographie (e.g. adsorption) ou la précipitation.

**La composition liquide ou le sirop (méth)acrylique** selon l'invention comprend un polymère (méth)acrylique (P1), un monomère (méth)acrylique (M1) ou un mélange de monomères (méth)acryliques, et un composé réticulant selon de formule (I). La composition liquide selon l'invention peut en outre comprendre au moins un amorceur radicalaire.

La viscosité dynamique de la composition liquide ou du sirop (méth)acrylique est dans une plage de 10 mPa*s à 10000 mPa*s, de préférence de 20 mPa*s à 7000 mPa*s et avantageusement de 20 mPa*s à 5000 mPa*s. La viscosité du sirop peut être aisément mesurée avec un rhéomètre ou un viscosimètre. La viscosité dynamique est mesurée à 25 °C. Si le sirop (méth)acrylique liquide présente un comportement newtonien, c'est-à-dire sans fluidification par cisaillement, la viscosité dynamique est indépendante du cisaillement dans un rhéomètre ou la vitesse du mobile dans un viscosimètre. Si la composition liquide présente un comportement non newtonien, c'est-à-dire avec fluidification par cisaillement, la viscosité dynamique est mesurée à un taux de cisaillement de $1s^{-1}$ à 25 °C.

La composition liquide ou le sirop (méth)acrylique selon l'invention, pour imprégner le substrat fibreux, comprend

en particulier un monomère (méth)acrylique ou un mélange de monomères (méth)acryliques, un polymère (méth)acrylique, composé réticulant selon de formule (I) selon l'invention et au moins au moins un amorceur radicalaire.

Une fois polymérisé, le monomère (méth)acrylique (M1) est transformé en polymère (méth)acrylique (P2) comprenant les motifs monomères de monomère (méth)acrylique (M1).

Le polymère thermoplastique (méth)acrylique possède une température de transition vitreuse (Tg) comprises entre 50°C et 160°C, de préférence comprises entre 70°C et 140 °C, et de manière encore plus préférée 90°C et 120 °C. De préférence, ces températures sont mesurées par Calorimétrie Différentielle à Balayage selon les conditions spécifiées dans les normes ISO 11357-2/2013 pour Tg et ISO 11357-3/2011 pour Tf.

En outre le polymère thermoplastique (méth)acrylique ou une partie du polymère thermoplastique (méth)acrylique possède un indice de fluage (MFI) selon l'ISO 1133 (230 °C/3,8 kg) inférieur à 20 g/10 min. De préférence, l'indice de fluage est inférieur à 18 g/10 min, plus préférablement inférieur à 16 g/10 min, avantageusement inférieur à 13 g/10 min.

**En ce qui concerne le monomère (méth) acrylique (M1)**, le monomère est choisi parmi l'acide acrylique, l'acide méthacrylique, des monomères alkylacryliques, des monomères alkylméthacryliques, des monomères hydroxyalkylacryliques et des monomères hydroxyalkylméthacryliques, et des mélanges de ceux-ci.

De préférence, le monomère (méth)acrylique (M1) est choisi parmi l'acide acrylique, l'acide méthacrylique, des monomères hydroxyalkylacryliques, des monomères hydroxyalkylméthacryliques, des monomères alkylacryliques, des monomères alkylméthacryliques et des mélanges de ceux-ci, le groupe alkyle contenant de 1 à 22 carbones linéaires, ramifiés ou cycliques ; le groupe alkyle contenant de préférence de 1 à 12 carbones linéaires, ramifiés ou cycliques.

Avantageusement, le monomère (méth)acrylique est choisi parmi les méthacrylate de méthyle, méthacrylate d'éthyle, acrylate de méthyle, acrylate d'éthyle, acide méthacrylique, acide acrylique, acrylate de n-butyle, acrylate d'isobutyle, méthacrylate de n-butyle, méthacrylate d'isobutyle, acrylate de cyclohexyle, méthacrylate de cyclohexyle, acrylate d'isobornyle, méthacrylate d'isobornyle, acrylate d'hydroxyéthyle et méthacrylate de hydroxyéthyle, et des mélanges de ceux-ci.

Selon un mode de réalisation préféré, au moins 50 % en poids et de préférence au moins 60 % en poids du monomère (méth)acrylique est du méthacrylate de méthyle.

Selon un premier mode de réalisation plus préféré, au moins 50 % en poids, de préférence au moins 60 % en poids, plus préférablement au moins 70 % en poids, avantageusement au moins 80 % en poids et encore plus avantageusement 90 % en poids du monomère est un mélange de méthacrylate de méthyle avec facultativement au moins un autre monomère.

**En ce qui concerne le polymère (méth)acrylique (P1),** il peut être mentionné des méthacrylates de polyalkyle ou des acrylates de polyalkyle. Selon un mode de réalisation préféré, le polymère (méth)acrylique est le poly(méthacrylate de méthyle) (PMMA).

Le terme « PMMA » désigne un homopolymère ou copolymère de méthacrylate de méthyle (MMA) ou des mélanges de ceux-ci.

Selon un mode de réalisation, l'homo- ou copolymère de méthacrylate de méthyle (MMA) comprend au moins 70 %, de préférence au moins 80 %, avantageusement au moins 90 % et plus avantageusement au moins 95 % en poids de méthacrylate de méthyle.

Selon un autre mode de réalisation, le PMMA est un mélange d'au moins un homopolymère et au moins un copolymère de MMA, ou un mélange d'au moins deux homopolymères ou deux copolymères de MMA avec un poids moléculaire moyen différent, ou un mélange d'au moins deux copolymères de MMA ayant une composition de monomères différente.

Le copolymère de méthacrylate de méthyle (MMA) comprend de 70 % à 99,7 % en poids de méthacrylate de méthyle et de 0,3 % à 30 % en poids d'au moins un monomère contenant au moins une insaturation éthylénique qui peut copolymériser avec le méthacrylate de méthyle.

Ces monomères sont bien connus et il peut être particulièrement mentionné les acides acrylique et méthacrylique et des (méth)acrylates d'alkyle dans lesquels le groupe alkyle contient de 1 à 12 atomes de carbone. À titre d'exemples, il peut être mentionné l'acrylate de méthyle et le (méth)acrylate d'éthyle, de butyle ou de 2-éthylhexyle. De préférence, le comonomère est un acrylate d'alkyle dans lequel le groupe alkyle contient de 1 à 4 atomes de carbone.

Selon un premier mode de réalisation préféré, le copolymère de méthacrylate de méthyle (MMA) comprend de 80 % à 99,7 %, avantageusement de 90 % à 99,7 % et plus avantageusement de 90 % à 99,5 % en poids de méthacrylate de méthyle et de 0,3 % à 20 %, avantageusement de 0,3 % à 10 % et plus avantageusement de 0,5 % à 10 % en poids d'au moins un monomère contenant au moins une insaturation éthylénique qui peut copolymériser avec le méthacrylate de méthyle. De préférence, le comonomère est choisi parmi l'acrylate de méthyle et l'acrylate d'éthyle, et des mélanges de ceux-ci.

Le poids moléculaire moyen en poids du polymère (méth)acrylique (P1) doit être élevé, c'est-à-dire supérieur à 50 000 g/mol et de préférence supérieur à 100 000 g/mol.

Le poids moléculaire moyen en poids peut être mesuré par chromatographie d'exclusion stérique (SEC).

Le polymère (méth)acrylique est totalement soluble dans le monomère (méth)acrylique ou dans le mélange

de monomères (méth)acryliques. Il permet que la viscosité du monomère (méth)acrylique ou du mélange de monomères (méth)acryliques soit augmentée. La solution obtenue est généralement appelée « sirop » ou « prépolymère ». La valeur de viscosité dynamique du sirop (méth)acrylique liquide est comprise entre 10 mPa.s et 10 000 mPa.s. La viscosité du sirop peut être aisément mesurée avec un rhéomètre ou un viscosimètre. La viscosité dynamique est mesurée à 25 °C.

Avantageusement, le sirop (méth)acrylique liquide ne contient aucun solvant additionnel ajouté volontairement.

**En ce qui concerne l'amorceur radicalaire, il peut être mentionné** des amorceurs de polymérisation radicalaires de préférence hydrosolubles ou des amorceurs de polymérisation radicalaires liposolubles ou partiellement liposolubles.

Les amorceurs de polymérisation radicalaires hydrosolubles sont notamment les persulfates de sodium, de potassium ou d'ammonium, utilisés seuls ou en présence d'agents réducteurs tels que les métabisulfites ou hydrosulfites de sodium, le thiosulfate de sodium, le formaldéhyde sulfoxylate de sodium, un mélange de sel disodique de 2-hydroxy-2-sulfinoacide acétique, de sulfite de sodium et de sel disodique de 2-hydroxy-2-sulfoacide acétique ou encore un mélange de sel disodique de l'hydroxysulfino d'acide acétique et de sel disodique de l'hydroxysulfo d'acide acétique.

Les amorceurs de polymérisation radicalaires liposolubles ou partiellement liposolubles sont notamment des peroxydes ou hydroperoxydes et des dérivés de 1 ' azobisisobutyronitrile. Les peroxydes ou hydroperoxydes sont utilisés en combinaison avec les agents réducteurs décrits précédemment de façon à baisser leur température d'activation.

Le pourcentage massique d'amorceur par rapport au poids total de mélange de monomères est de préférence compris entre 0,05% en poids et 3% en poids, de préférence entre 0,1% en poids et 2% en poids.

De façon préférée, la composition liquide (méth)acrylique comporte au moins deux amorceurs radicalaires dont au moins un est activé par la chaleur.

La composition liquide selon l'invention peut comprendre en outre, de façon optimale, un activateur pour la polymérisation.

L'activateur ou accélérateur de polymérisation est choisi parmi des amines tertiaires telles que la N,N-diméthyl-p-toluidine (DMPT), la N,N-dihydroxyéthyl-p-toluidine (DHEPT), des catalyseurs à métal de transition solubles en milieu organique ou des mélanges de ceux-ci.

La composition liquide peut comprendre en outre **un ou plusieurs additifs et/ou une ou plusieurs charges**, tels que charges carbonées, les charges minérales et les additifs organiques. L'ensemble des additifs et charges facultatifs sont ajoutés au sirop (méth)acrylique liquide avant l'imprégnation et/ou la polymérisation.

Les **charges carbonées** peuvent être en particulier du charbon actif, de l'anthracite naturel, de l'anthracite synthétique, du noir de carbone, du graphite naturel, du graphite synthétique, des nanocharges carbonées ou leurs mélanges. Elles sont de préférence choisies parmi des nanocharges carbonées, en particulier des graphènes et/ou des nanotubes de carbone et/ou des nanofibrilles de carbone ou leurs mélanges. Ces charges permettent de conduire l'électricité et la chaleur, et permettent par conséquent d'améliorer la lubrification de la matrice polymère lorsqu'elle est chauffée. Elles peuvent permettre alors une réduction accrue des temps de cycle ou faciliter l'assemblage, l'ajustement ou la réparation sur le site d'installation.

Les **charges minérales** comprennent notamment les hydroxydes métalliques, qui se présentent plus particulièrement sous forme de trihydate d'alumine ($Al(OH)_3$) ou d'hydroxyde de magnésium (Mg(OH)) ou oxyde de magnésium (MgO), les hydroxides de calcium et les charges minérales telles que le carbonate de calcium, le dioxyde de titane ou la silice ou les nanocharges minérales telles que les nanodioxide de titane ou les nanosilices.

En tant **qu'additifs,** il est possible de mentionner les additifs organiques tels que les modificateurs de la résistance aux impacts (modifiant chocs) ou les copolymères séquencés, les stabilisateurs thermiques, les stabilisateurs UV, les lubrifiants, modificateurs de viscosité, les modificateurs de pH (soude), les modificateurs de granulométrie (sulfate de sodium), les biocides, et leurs mélanges. Ces additifs permettent d'améliorer notamment les propriétés rhéologiques, chimiques et d'adhérence de la matrice de polymère thermoplastique (méth)acrylique. Le modifiant choc est sous forme de particules fines ayant un noyau élastomère et au moins une enveloppe thermoplastique, la taille des particules étant en général inférieure à 1 $\mu$m et avantageusement de 50 à 300 nm. Le modificateur de résistance aux chocs est préparé par polymérisation en émulsion. La proportion de modifiants chocs dans la matrice thermoplastique polymère est de 0 à 50 %, de préférence de 0 à 25 %, et avantageusement de 0 à 20 % en poids.

Le pourcentage massique de l'ensemble des additifs et des charges par rapport au poids total de matrice de polymère thermoplastique (méth)acrylique est de préférence inférieur à 30%, de préférence inférieur à 10%.

Avantageusement, la composition (méth)acrylique liquide ne contient aucun catalyseur à base de métal. Aucun additif comprenant un métal en tant qu'activateur pour accélérer de façon catalytique la réaction de polymérisation n'est ajouté à la composition (méth)acrylique liquide selon l'invention. Cela concerne, en particulier, des composés à base d'étain tels que le chlorure d'étain.

La teneur de l'activateur par rapport au monomère (méth)acrylique (M1) de la composition (méth)acrylique liquide est de 100 ppm à 10000 ppm (en poids), de préférence de 200 ppm à 7000 ppm en poids et avantageusement, de 300 ppm à 4000 ppm.

Afin de conserver une viscosité dynamique de la composition liquide ou du sirop (méth)acrylique, outre le fait

que cela permette une bonne imprégnation du substrat fibreux, si nécessaire, et de conserver les propriétés thermoplastiques de la matrice obtenue après polymérisation du substrat fibreux pré-imprégné avec le sirop, **les composés du sirop sont incorporés dans les pourcentages en masse suivants :**

Le(s) monomère(s) (méth)acrylique(s) (M1) dans la composition liquide ou le sirop (méth)acrylique sont présents dans proportions comprises entre 40 % et 90 % en poids et de préférence entre 45 % et 85 % en poids de la composition comprenant le(s) monomère(s) (méth)acrylique(s) (M1) et le polymère (méth)acrylique (P1).

Le(s) polymère(s) (méth)acrylique(s) (P1) dans la composition liquide ou le sirop (méth)acrylique sont présents dans une proportion d'au moins 5 % et avantageusement au moins 10 % en poids de la composition comprenant le (s) monomère(s) (méth)acrylique(s) (M1) et le polymère (méth)acrylique (P1).

Le(s) polymère(s) (méth)acrylique(s) (P1) dans le sirop (méth)acrylique liquide sont présents dans une proportion de pas plus de 50 % en poids, de préférence pas plus de 40 % et avantageusement pas plus de 30 % en poids de la composition comprenant le(s) monomère(s) (méth)acrylique(s) (M1) et le polymère (méth)acrylique (P1) .

L'ensemble des additifs et charges facultatifs sont ajoutés au sirop (méth)acrylique liquide avant l'imprégnation et/ou la polymérisation.

Avantageusement, la quantité de composé réticulant selon l'invention dans la composition liquide est inférieure à 10 phr, de préférence inférieure à 5 phr, par rapport à la somme du monomère (méth)acrylique (M1) et du polymère (méth)acrylique (P1). En effet, une telle proportion permet d'améliorer la réversibilité de la réaction de Diels-Alder au sein du polymère thermoplastique réticulé. La quantité de composé réticulant dans la composition liquide selon l'invention est supérieure à 0,5 phr, par rapport à la somme du monomère (méth)acrylique (M1) et du polymère (méth)acrylique (P1). Ces concentrations pourront être ajustées en fonction du nombre de fonction époxyisoindole portées par le composé réticulant ainsi qu'en fonction du groupement L sélectionné.

Avantageusement, la composition liquide comprend en outre du hydroxyéthylméthacrylate permettant d'améliorer la solubilité du composé réticulant dans la composition liquide.

En ce qui concerne **le procédé de fabrication de la composition liquide ou du sirop (méth)acrylique**, ce dernier comporte une première étape de mélange d'un composé réticulant selon l'invention à un sirop comprenant le monomère (méth)acrylique (M1) ou un mélange de monomères (méth)acryliques et/ou au moins un polymère (méth)acrylique (P1).

Cette première étape permet d'améliorer la répartition du composé réticulant dans la composition à polymériser. En outre, de façon préférée, cette première étape permet de solubiliser le composé réticulant selon l'invention.

De façon préférée, la première étape consiste à préparer un premier sirop comprenant le monomère (méth)acrylique (M1) ou un mélange de monomères (méth)acryliques et le composé réticulant selon l'invention.

En effet, le composé réticulant peut être retrouvé dans la composition liquide sous la forme d'une suspension homogène ou sous forme solubilisée. La forme solubilisée est préférée car elle permet une meilleure réversibilité de la réaction de Diels-Alder et donc une meilleure dé-réticulation du polymère thermoplastique formé à partir de la composition liquide.

De façon préférée, le procédé de fabrication de la composition liquide selon l'invention comporte une seconde étape consistant à ajouter au moins un polymère (méth)acrylique (P1) au mélange préparé dans l'étape précédente.

De façon particulière, le procédé de fabrication de la composition liquide selon l'invention comporte une troisième étape correspondant à l'ajout d'au moins un amorceur radicalaire.

L'invention porte en outre sur l'**utilisation de la composition liquide** selon l'invention **pour fabriquer des formulations** pour les arts graphiques (e.g. encre pour impression jet d'encre, vernis de surimpression, encre d'écran, encre lithographique, encre pour flexographie), pour des revêtements (e.g. revêtements de fibres optiques, de bois, de métal, de plastique, dans les produits cosmétiques), pour des adhésifs (tels que des adhésifs structuraux), pour des peintures (telles que des peintures routières), pour l'étanchéité de toits ou de sols, pour des gelcoats ou topcoat, (pour une utilisation par exemple dans l'automobile, la marine, les éoliennes, les marbres artificiels), pour des chevilles chimiques ou des renforts pour ciment.

L'invention porte en outre sur **l'utilisation de la composition liquide** selon l'invention pour **fabriquer des pièces thermoplastiques** ou **fabriquer des pièces composites** ainsi que sur les procédés de fabrication associés.

Ainsi, de façon particulière, l'invention porte sur un **procédé de fabrication de pièces thermoplastiques** comprenant les étapes de placer la composition liquide selon l'invention ou préparée selon le procédé de préparation selon l'invention dans des moyens de polymérisation, et d'initier la polymérisation.

Alternativement, de façon particulière, l'invention porte sur un **procédé de fabrication de pièces composites** comprenant les étapes d'imprégnation de fibres ou d'un substrat fibreux avec la composition liquide selon l'invention ou préparée selon le procédé de préparation selon l'invention dans des moyens de polymérisation, et d'initier la polymérisation.

De façon préférée, l'étape de polymérisation est conduite à une température inférieure à 200 °C, de préférence inférieure à 150 °C. Cela permet de ne pas initier de réaction de rétro Diels-Alder et ainsi permet d'obtenir rapidement un polymère réticulé.

**En ce qui concerne le procédé d'imprégnation les fibres ou du substrat fibreux,** celui-ci comprend une étape d'imprégnation du substrat fibreux avec la composition liquide ou le sirop (méth)acrylique.

Cette étape d'imprégnation peut être conduite dans un moule ou un moule fermé ou un bain.

Si la viscosité du sirop (méth)acrylique liquide à une température donnée est légèrement trop élevé pour le procédé d'imprégnation, il est possible de chauffer le sirop de manière à obtenir un sirop plus liquide pour un mouillage suffisant et une imprégnation correcte et complète du substrat fibreux.

**En ce qui concerne le substrat fibreux**, il peut être mentionné plusieurs fibres, des stratifils unidirectionnels ou un mat à filament continu, des tissus, des feutres ou des non-tissés qui peuvent être sous la forme de bandes, nappes, tresses, mèches ou pièces. Le matériau fibreux peut avoir différentes formes et dimensions, unidimensionnelles, bidimensionnelles ou tridimensionnelles. Un substrat fibreux comprend un assemblage d'une ou plusieurs fibres. Lorsque les fibres sont continues, leur assemblage forme des tissus.

La forme unidimensionnelle correspond à des fibres longues linéaires. Les fibres peuvent être discontinues ou continues. Les fibres peuvent être agencées de façon aléatoire ou parallèle les unes aux autres, sous la forme d'un filament continu. Une fibre est définie par son facteur de forme, qui est le rapport entre la longueur et le diamètre de la fibre. Les fibres utilisées dans la présente invention sont des fibres longues ou des fibres continues. Les fibres présentent un facteur de forme d'au moins 1000, de préférence au moins 1500, plus préférablement au moins 2000, avantageusement au moins 3000 et plus avantageusement au moins 5000, encore plus avantageusement au moins 6000, encore plus avantageusement au moins 7500 et le plus avantageusement au moins 10 000.

La forme bidimensionnelle correspond à des mats ou des renforcements fibreux non-tissés ou tissés ou des faisceaux de fibres, qui peuvent également être tressés. Même si la forme bidimensionnelle a une certaine épaisseur et, par conséquent, en principe une troisième dimension, elle est considérée comme étant bidimensionnelle selon la présente invention.

La forme tridimensionnelle correspond, par exemple, à des mats ou des renforcements fibreux non-tissés ou des faisceaux empilés ou pliés de fibres ou des mélanges de ceux-ci, un assemblage de la forme bidimensionnelle dans la troisième dimension.

Les origines du matériau fibreux peuvent être naturelles ou synthétiques. En tant que matériau naturel, on peut mentionner des fibres végétales, des fibres de bois, des fibres animales ou des fibres minérales.

Des fibres naturelles sont, par exemple, des fibres de sisal, jute, chanvre, lin, coton, noix de coco, et des fibres de banane. Des fibres animales sont, par exemple, de la laine ou des poils.

En tant que matériau synthétique, il peut être mentionné des fibres polymères choisies parmi des fibres de polymères thermodurcissables, de polymères thermoplastiques ou des mélanges de ceux-ci.

Les fibres polymères peuvent être constituées de polyamide (aliphatique ou aromatique), polyester, alcool polyvinylique, polyoléfines, polyuréthanes, chlorure de polyvinyle, polyéthylène, polyesters insaturés, résines époxy et esters vinyliques

Les fibres minérales peuvent également être choisies parmi des fibres de verre, en particulier de type E, R ou S2, des fibres de carbone, des fibres de bore ou des fibres de silice.

Le substrat fibreux de la présente invention est choisi parmi des fibres végétales, des fibres de bois, des fibres animales, des fibres minérales, des fibres polymères synthétiques, des fibres de verre et des fibres de carbone, et des mélanges de celles-ci.

De préférence, le substrat fibreux est choisi parmi des fibres minérales.

Les fibres du substrat fibreux ont un diamètre compris entre 0,005 $\mu$m et 100 $\mu$m, de préférence entre 1 $\mu$m et 50 $\mu$m, plus préférablement entre 5 $\mu$m et 30 $\mu$m et avantageusement, entre 10 $\mu$m et 25 $\mu$m.

De préférence, les fibres du substrat fibreux de la présente invention sont choisies parmi des fibres continues (ce qui signifie que le facteur de forme ne s'applique pas nécessairement comme pour des fibres longues) pour la forme unidimensionnelle, ou pour des fibres longues ou continues pour la forme bidimensionnelle ou tridimensionnelle du substrat fibreux.

Selon un autre aspect additionnel, l'invention concerne un matériau composite polymère comprenant une matrice (méth)acrylique thermoplastique et un substrat fibreux utilisé en tant que renforcement, dans lequel le substrat fibreux est constitué de fibres longues, ledit matériau composite étant caractérisé en ce que la matrice (méth)acrylique thermoplastique est obtenue après polymérisation dudit substrat fibreux pré-imprégné avec ladite composition liquide selon l'invention ou le sirop (méth)acrylique.

Un autre aspect de la présente invention est un procédé de fabrication de pièces thermoplastiques, comprenant les étapes suivantes :

i) placement de la composition liquide ou du sirop (méth)acrylique dans des moyens de polymérisation, et,
ii) polymérisation de la composition liquide ou du sirop (méth)acrylique.

Un autre aspect de la présente invention est un procédé de fabrication de pièces ou produits mécaniques ou

structurés, comprenant les étapes suivantes :

i) imprégnation d'un substrat fibreux avec la composition liquide ou sirop (méth)acrylique selon l'invention,
ii) polymérisation de la composition liquide ou du sirop (méth)acrylique imprégnant ledit substrat fibreux.

En ce qui concerne **le procédé de fabrication de pièces composites, mais également de pièces ou produits mécaniques ou structurés,** différents procédés peuvent être utilisés pour fabriquer ces pièces. Il peut être mentionné l'infusion de résine assistée par le vide (VARI), la pultrusion, le moulage par infusion sous vide, le moulage par infusion sous pression, le moulage à l'autoclave, le moulage par transfert de résine (RTM) et des variantes de celui-ci telles que (HP-RTM, C-RTM, I-RTM), le moulage par réaction-injection (RIM), le moulage par réaction-injection renforcée (R-RIM) et des variantes de celui-ci, le moulage à la presse, le moulage par compression, le moulage par compression de liquide (LCM) ou le moulage en feuille (SMC) ou le moulage en vrac (BMC).

Un premier procédé de fabrication préféré pour fabriquer des pièces composites est un procédé selon lequel la composition liquide est transférée sur le substrat fibreux par imprégnation du substrat fibreux dans un moule. Les procédés nécessitant un moule sont énumérés ci-dessus et comprennent le mot moulage.

Un deuxième procédé de fabrication préféré pour fabriquer des pièces composites sont des procédés selon lesquels la composition liquide est utilisée dans le procédé de pultrusion. Les fibres sont guidées vers une filière chaude où une résine thermoplastique comportant la composition liquide selon l'invention est injectée. Les fibres sous forme de substrat fibreux sont, par exemple, sous la forme d'un stratifil unidirectionnel ou un mat de filament continu. Après imprégnation dans le lot de résine, les fibres mouillées sont tirées à travers une filière chauffée, où la polymérisation se produit.

Un troisième procédé de fabrication préféré est l'infusion de résine assistée par le vide (VARI).

Le procédé de fabrication de pièces composites, mais également de pièces ou produits mécaniques ou structurés, peut comprendre en outre l'étape de post-formage. Le post-formage comprend une flexion ainsi qu'une modification de la forme de la pièce composite.

Le procédé de fabrication de pièces composites, mais également de pièces ou produits mécaniques ou structurés, peut comprendre en outre l'étape de soudage ou collage ou laminage.

Les pièces thermoplastiques obtenues par les procédés selon l'invention peuvent être post-formées après polymérisation de la composition liquide de l'invention. Le formage comprend une flexion ainsi qu'une modification de la forme de la pièce composite.

Les pièces thermoplastiques ou pièces composites fabriquées obtenues après polymérisation de la composition liquide de l'invention et/ou par les procédés selon l'invention peuvent être soudées, collées ou laminées.

En ce qui concerne l'utilisation des pièces mécaniques constituées de matériau composite fabriquées ainsi, il peut être mentionné des applications dans l'automobile, des applications dans le transport telles que les bus ou les camions, des applications nautiques, des applications ferroviaires, le sport, des applications aéronautiques et aérospatiales, des applications photovoltaïques, des applications informatiques, des applications dans la construction et le bâtiment, des applications dans les télécommunications et des applications dans l'énergie éolienne.

La pièce mécanique constituée de matériau composite est en particulier, une pièce de véhicule à moteur, une pièce de bateau, une pièce de bus, une pièce de train, un article de sport, une pièce d'avion ou d'hélicoptère, une pièce d'engin spatial ou de fusée, une pièce de module photovoltaïque, un matériau pour la construction ou le bâtiment, par exemple des armatures, chevilles et étriers composites pour le génie civil et la construction à grande hauteur, une pièce de turbine éolienne, par exemple une semelle de longeron de poutrelle de pale de turbine éolienne, une pièce de mobilier, une pièce de construction ou de bâtiment, une pièce de téléphone ou de téléphone cellulaire, une pièce d'ordinateur ou de télévision, ou une pièce d'imprimante ou de photocopieuse.

Dans un premier mode de réalisation préféré, la pièce mécanique constituée de matériau composite est, en particulier, un matériau pour la construction ou le bâtiment, par exemple des armatures, chevilles et étriers composites pour le génie civil et la construction à grande hauteur.

Dans un deuxième mode de réalisation préféré, la pièce mécanique constituée de matériau composite est, en particulier, une pièce de turbine éolienne, par exemple, une semelle de longeron de poutrelle de pale de turbine éolienne.

Dans un troisième mode de réalisation préféré, la pièce mécanique constituée de matériau composite est une pièce structurelle automobile, telle qu'un plancher, un pied milieu, une traverse de porte, un renfort latéral, une traverse de siège, un bouclier avant.

## [Exemples]

### Première étape : préparation d'un composé réticulant

La synthèse du composé réticulant a été effectuée avec différents ratios de composés diéne conjugué (e.g.

méthacrylate de furfuryle - FMA) et diénophile (e.g. bismaléimide). Les réactifs sont introduits dans un ballon contenant du tétrahydrofurane (THF) anhydre. Le mélange réactionnel est ensuite placé sous agitation et atmosphère inerte puis le spectre RMN 1H du mélange réactionnel de départ est suivi.

Comme présenté à la figure 1, l'augmentation de la quantité de FMA ajouté dans le milieu réactionnel favorise la réaction d'addition Diels-Alder. En effet, pour un ratio équimolaire théorique, la réaction évolue très rapidement dès les premières heures mais tend à stagner et évoluer très lentement après 10 heures pour atteindre un taux de conversion n'étant pas supérieur à 75%. Pourtant, il est particulièrement avantageux que l'intégralité des fonctions imides soit additionnée de façon à améliorer la réticulation ultérieure du polymère du polymère thermoplastique. Ainsi, il est particulièrement avantageux d'utiliser un large excès de FMA afin de favoriser davantage la réaction entre le diénophile et les diènes portés par le FMA.

Après avoir obtenu un taux de conversion maximal au bout de 48h, le mélange réactionnel est purifié afin d'ôter l'excès de FMA présent. La purification a lieu par précipitation dans du méthanol anhydre glacé puis filtration sur Büchner. Le produit obtenu est resolubilisé dans un minimum de solvant THF anhydre puis re-précipité dans du méthanol. Le précipité est laissé sous agitation magnétique dans le solvant pendant 30 min afin d'extraire les dernières traces de FMA. Après filtration, le produit est séché sous vide à 25 °C toute une nuit afin d'extraire les dernières traces de méthanol.

Alternativement, le procédé de synthèse comporte une première étape de synthèse effectuée à partir du FMA mis en présence d'hydroxymaléimide avec un ratio molaire respectif de 4 pour 1. En effet, il a été montré qu'un excès de FMA conduisait à un rendement de réaction plus élevé (≈ 98%). La réaction a lieu en voie solvant dans du THF anhydre, sous agitation et atmosphère inerte à 60°C pendant 48 heures. Le composé obtenu est précipité à deux reprises dans du cyclohexane puis après filtration, il est séché quelques heures sous vide à 50°C. Une deuxième étape de synthèse destinée à former le composé réticulant a été réalisée par la réaction entre le monoalcool Diels-Alder préparé précédemment et un composé comportant une fonction isocyanate. Les deux réactifs sont introduits en stoechiométrie en présence de THF à hauteur de 10% en masse de réactifs. Un catalyseur, le dilaurate de dibutylétain - DBTDI est incorporé à raison de 0,15% en masse. La solution est ensuite agitée 24 h à 50°C sous atmosphère inerte. Le produit de réaction obtenu est ensuite précipité dans du cyclohexane. Après filtration sur Büchner, le produit est séché plusieurs heures sous vide à 50°C.

**Deuxième étape : préparation d'une composition liquide ou du sirop (méth)acrylique selon l'invention**

Une composition liquide est préparée par dissolution de 25 % en poids de PMMA (e.g. un copolymère de MMA comprenant de l'acrylate d'éthyle en tant que comonomère) en tant que (P1) dans 75 % en poids de méthacrylate de méthyle en tant que (M1). À cette composition liquide est ajouté un composé réticulant et un amorceur radicalaire.

En fonction de la formulation de la composition liquide, le composé réticulant peut être soluble ou bien former une suspension homogène.

**Troisième étape : polymérisation d'une composition liquide ou du sirop (méth)acrylique**

Différentes proportions du composé réticulant sont ajoutées à la formulation P1 M1, respectivement 0.5phr, 2phr, 5phr et 10phr par rapport à la quantité de sirop utilisé. La polymérisation est ensuite réalisée dans un tube en verre de 5mm de diamètre régulé en température à l'aide un module chauffant. Les compositions selon l'invention respectives sont chauffées à une température modérée ($60<T°C<80$) dans une presse métallique sous une pression de 10 bar.

La synthèse des copolymères est effectuée par voie radicalaire à partir de sirops basse viscosité dont les cinétiques de polymérisation montrent une polymérisation rapide (<4min) à température modérée ($60<T°C<80$):

Comme cela est présenté en figure 2, l'ajout du composé réticulant selon l'invention n'a aucune influence sur la polymérisation des différents sirops. Cela nous permet ainsi de conserver des cinétiques rapides avec des taux de conversion, suivi par spectrométrie proche infrarouge, de près de 100 % atteint après des durées de polymérisation inférieures à 3-4min.

**Evaluation des performances**

L'évaluation du taux de gonflement

L'évaluation du taux de gonflement apporte une information supplémentaire sur la création d'un réseau réticulé mais également une indication sur la densité de réticulation. En effet, on peut supposer qu'un réseau plus dense aura tendance à avoir un taux de gonflement plus faible. Pour cela, des échantillons de masse ($m_{ini}$) sont introduits dans quelques millilitres de THF pendant 72h.

Après cette période, la partie insoluble est filtrée puis pesée, correspondant alors au réseau gonflé ($m_{rg}$). Le

THF contenant la fraction soluble est placé dans un évaporateur rotatif (rotavapor) de manière à déterminer la masse de matière organique solubilisée ($m_{sol}$). Le taux de gonflement est ensuite défini de la façon suivante:

$$\text{Taux de gonflement (\%)} = \frac{m_{rg}}{m_{ini}-m_{sol}} \times 100$$

Ces analyses ont montré que plus le pourcentage de composé réticulant augmente et moins le taux de gonflement du polymère obtenu est important. Ainsi, avec une quantité de composé supérieure à 0.5 phr, il y a formation d'un réseau tridimensionnel dont on peut faire varier la densité de réticulation en faisant varier la concentration de composé réticulant. Par exemple, le taux de gonflement obtenu avec 2 phr peut être d'environ 400 % et d'environ 320 % avec 5 phr de composé réticulant. De la même façon, on peut observer que pour une quantité trop faible du composé réticulant (0,5 phr et inférieur), le polymère obtenu comporte un réseau réticulé mais ce dernier n'est pas un réseau suffisamment dense et finit par se désagréger et se solubiliser dans le solvant.

Thermo-réversibilité de la réaction Diels-Alder

Le composé réticulant est mis en solution dans du diméthylformamide deutéré puis introduit dans un tube RMN de 5mm. La réaction rétro DA peut être alors suivie *in situ* par spectroscopie RMN 1H à différentes températures.

Les thermoplastiques ont la propriété d'avoir un seuil d'écoulement ainsi que la capacité à se solubiliser au contact d'un solvant adapté. Les polymères thermodurcissables quant à eux ne peuvent ni s'écouler ni se solubiliser. De ce fait, sachant que la réaction Diels-Alder est particulièrement sensible à la température, il nous est impossible d'utiliser les propriétés d'écoulement comme facteur déterminant. Par conséquent, c'est en évaluant la solubilité des pseudo-thermoplastique en milieu solvant qu'il est possible de déterminer la réversibilité ou non des copolymères obtenus. Pour cela, chaque copolymère synthétisé est plongé dans du THF (solvant du PMMA) afin de déterminer dans un premier temps l'obtention ou non d'un réseau tridimensionnel (étude de gonflement). Dans un second temps, après avoir subi l'effet rétro Diels-Alder, le caractère réversible ou non est déterminé en testant à nouveau sa solubilité.

Lorsque l'on chauffe un produit d'addition DA celui-ci retourne à son état initial où l'on retrouve d'un côté le BMI et de l'autre le furfuryl méthacrylate. Ceci est confirmé dans la figure 3 présentant les résultats de suivi par RMN à 100°C.

Les suivis cinétiques de la réaction rétro DA permettent de confirmer que l'aspect réversible de l'addition Diels-Alder dépend fortement de la température. En effet, comme on peut le constater, plus la température est élevée et plus le taux de conversion rétro DA évolue fortement dès les premiers instants. Ceci est encore plus vrai à partir de 120°C où dès les premières minutes, la quasi intégralité des composés réticulants se sont dissociés au profit de la reformation du BMI et FMA initiaux.

On peut également noter qu'à partir d'une certaine quantité de composé réticulant, les différents copolymères semblent présenter un caractère réversible réduit. C'est le cas à partir d'une addition de 5phr pour un composé réticulant soluble dans le sirop liquide et d'une addition de 10phr de composé réticulant en suspension homogène dans le sirop liquide. En effet, même après un avoir atteint la température de rétro Diels-Alder, ces formulations restent au moins partiellement insolubles dans le THF et conservent un aspect de polymère gonflé. En présence d'un mélange initial miscible, on peut penser que les points de réticulation sont répartis de façon homogène au sein du polymère tandis qu'ils peuvent être plus centralisés dans le cas d'un mélange hétérogène (suspension). Lorsque le composé réticulant forme une suspension homogène, sa solubilité peut être améliorée par l'ajout d'hydroxyéthylméthacrylate dans la composition liquide.

Des études basent leurs concepts de polymères réversibles sur la réaction de deux composés, l'un étant constitué de fonctions furanes pendantes et autre étant un bismaleimide. Néanmoins, cette étude propose la synthèse d'un unique composé simple et efficace. En effet, lors de la mise en oeuvre de composés multiples, en plus d'ajouter une contrainte liée à la préparation du mélange diène confugué + diènophile, le résultat de l'addition Diels-Alder ne peut pas être correctement maitrisé notamment vis-à-vis de sa cinétique. Comme on a pu le voir, celle-ci peut être très longue et de plus fortement dépendante de la température.

L'avantage ici d'utiliser directement un composé réticulant réside dans le fait que toutes les clés nécessaires à l'obtention d'un réseau réversible se trouvent sous la forme d'un seul produit, stable et simple d'utilisation. De plus, l'intégralité des molécules possèdent un site réversible prêt à l'emploi offrant donc une efficacité maximale. En outre, nous avons pu voir que ce type de comonomère était parfaitement capable de s'adapter à un système de polymérisation rapide ouvrant ainsi un large panel d'applications. La polymérisation de ce type de composé est avantageusement réalisée à une température inférieure à la température rétro DA. L'obtention d'un réseau thermoréversible permet également au polymère obtenu d'améliorer sa résistance aux attaques chimiques et notamment aux solvants mais permet aussi d'augmenter sa tenue en température.

En outre, l'utilisation d'une composition liquide selon l'invention nous donne un mélange réactionnel de basse viscosité ce qui ouvre ce système à de nombreux procédés tels que le RTM ou l'infusion mais également différentes applications comme par exemple les formulations adhésives. En effet, on pourrait de cette façon assembler des pièces par collage puis en atteignant la température de rétro DA, on pourrait facilement les désassembler. De la même façon, il est possible de mettre en oeuvre cet état réversible dans les systèmes pré imprégnés, SMC, etc ... où la réaction DA pourrait conduire à un système B-stage puis lors du moulage permettrait l'écoulement grâce à effet rétro puis la polymérisation finale grâce à un système haute température.

## Revendications

**1.** Composition liquide comprenant :

a. au moins 5 % en poids et pas plus de 50 % en poids d'un polymère (méth)acrylique (P1),
b. entre 40 % et 90 % en poids d'un monomère (méth)acrylique (M1) ou un mélange de monomères (méth)acryliques, et
c. supérieure à 0,5 phr et inférieure à 10 phr, par rapport à la somme du monomère (méth)acrylique (M1) et du polymère (méth)acrylique (P1),d'un composé réticulant de formule (I)

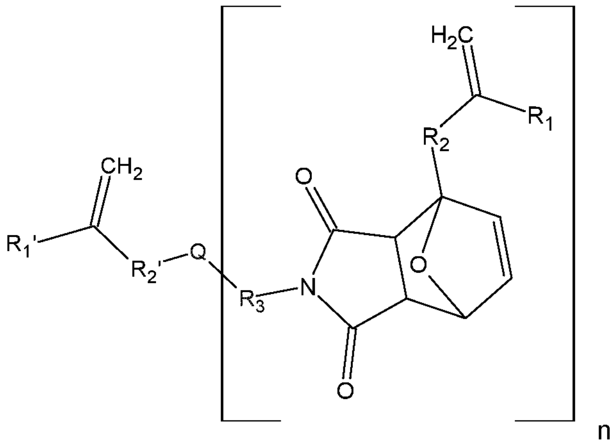

(I)

dans laquelle :

le groupement Q représente -Z-L- où :
le groupement Z représente une simple liaison ou

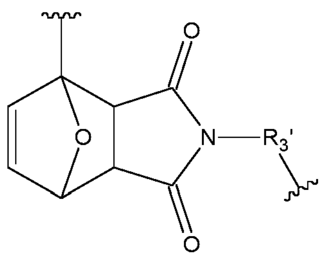

,

le groupement L représente une simple liaison ou un groupe sélectionné parmi : - $(C_1\text{-}C_6)$alkyle-, - $(C_2\text{-}C_6)$alkényle-, -$(C_2\text{-}C_6)$alkynyle-, - $(CONHR_4NHCOOR_5O)_m$-, - $(O\text{-}CO\text{-}NR_6)_m$-, - $(ORCHOHR_5)_m$-, - $(CH_2CHOHCH_2OR_4O)_m$-, - $(OR_4)_m$-, - $((CHOR_6)CH_2O\text{-}R_4)_m$-, - $(R_4\text{-}COO\text{-}R_5)_m$-, - $NR_6$-, -$SO_2$-, -$SO_2NR_6$-, -O-, -S-, -$CONR_6$-, -aryle-, -(aryle-$R_4)_m$-, - (hétéroaryle-$R_4)_m$-, -(($C_3\text{-}C_8$) hétérocyclyle-$R_4)_m$-, - (($C_3\text{-}C_{14}$)cycloalkyle-$R_4)_m$-, - $(C_1\text{-}C_6)$alkyle-$R_7$-$(C_1\text{-}C_6)$alkyle-, -$(C_2\text{-}C_6)$alkényle-$R_7$-$(C_1\text{-}C_6)$alkyle-, - $(C_1\text{-}C_6)$alkyle-$R_7$-$(C_2\text{-}C_6)$alkényle-,
ou - $(C_2\text{-}C_6)$alkényle-$R_7$-$(C_2\text{-}C_6)$alkényle-,

où les groupes $R_4$ et $R_5$ sont identiques ou différents et représentent un groupe sélectionné parmi : simple liaison, $-(C_1-C_6)$alkyle-, $-(C_2-C_6)$alkényle-, $-NR_6-$, $-SO_2-$, $-SO_2NR_6-$, -O-, -S-, $-CONR_6-$, -aryle-, -(aryle-$R_6)_m$-, - (hétéroaryle-$R_6)_m$-, -(($C_3-C_8$) hétérocyclyle)-$R_6)_m$-, -(($C_3-C_{14}$)cycloalkyle-$R_6)_m$-aryle-, -hétéroaryle-, -($C_3-C_8$) hétérocyclyle-, -($C_3-C_{14}$)cycloalkyle-, -($C_1-C_6$)alkyle-OCO-, ou $-CH_2-(CHOR_3)CH_2O-(C_1-C_6)$alkyle- ;

où $R_6$ représente un groupe sélectionné parmi : atome d'hydrogène, -($C_1-C_6$)alkyle, -($C_2-C_6$)alkényle, $-NH_2$, -COOH, $-SO_2H$, -OH, -SH, -aryle, -hétéroaryle, -($C_3-C_8$)hétérocyclyle, ou -($C_3-C_{14}$)cycloalkyle ;

où $R_7$ représente un groupe sélectionné parmi : simple liaison, $-(CONHR_4NHCOOR_5O)_m-$, $-(O-CO-NR_4)_m-$, $-(COR_4)_m-$, $-(OR_4)_m-$, $-(CH_2CHOHCH_2OR_4O)_m-$, $-((CHOR_4)CH_2O-R_5)_m-$, $-(R_4-COO-R_5)_m-$, $-NR_6-$, $-SO_2-$, $-SO_2NR_6-$, -O-, -S-, $-CONR_6-$, -aryle-, -(aryle-$R_4)_m$-, - (hétéroaryle-$R_4)_m$-, -(($C_3-C_8$)hétérocyclyle)-$R_4)_m$-, -(($C_3-C_{14}$)cycloalkyle-$R_4)_m$-, -($C_1-C_6$)alkyle-OCO-, ou $-CH_2-(CHOR_3)CH_2O-(C_1-C_6)$alkyle- ;

les groupements $R_1$ et $R_1$' sont identiques ou différents et représentent un atome d'hydrogène ou un groupe alkyle à chaine linéaire, ou ramifiée contenant jusqu'à 6 atomes de carbone ; les groupements $R_2$ et $R_2$' sont identiques ou différents et représentent une simple liaison ou un groupe sélectionné parmi : -($C_1-C_6$)alkyle-, -($C_2-C_6$)alkényle-, -COO-, ou COO-($C_1-C_6$)alkyle-, de préférence -COO- ou COO-($C_1-C_6$)alkyle- ;

les groupements $R_3$ et $R_3$' sont identiques ou différents et représentent -NHCOO- ;

avec n, représentant le nombre d'unités de répétition, compris entre 1 et 10,

avec m, représentant le nombre d'unités de répétition, compris entre 1 et 20, par exemple entre 1 et 10.

2. Composition liquide selon la revendication 1 **caractérisé en ce que** le composé réticulant est choisi dans le groupe constitué des composés de formule (Ia)

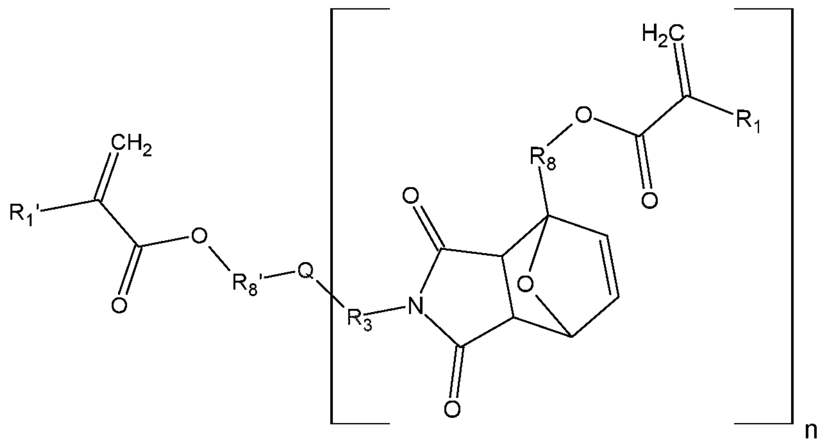

(Ia)

dans laquelle :

les groupements $R_8$ et $R_8$' sont identiques ou différents et représentent une simple liaison ou un groupe alkyle à chaine linéaire, ou ramifiée contenant jusqu'à 6 atomes de carbone.

3. Composition liquide selon l'une des revendications 1 ou 2 **caractérisé en ce que** le composé réticulant est choisi dans le groupe constitué des composés de formule (Ib)

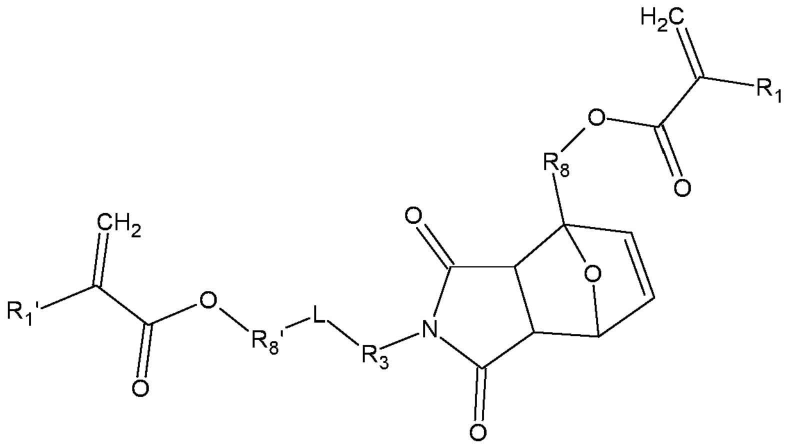

(Ib)

dans laquelle :
les groupements $R_8$ et $R_8$' sont identiques ou différents et représentent une simple liaison ou un groupe alkyle à chaine linéaire, ou ramifiée contenant jusqu'à 6 atomes de carbone.

4. Composition liquide selon l'une des revendications 1 ou 2 **caractérisé en ce que** le composé réticulant est choisi dans le groupe constitué des composés de formule (Ic)

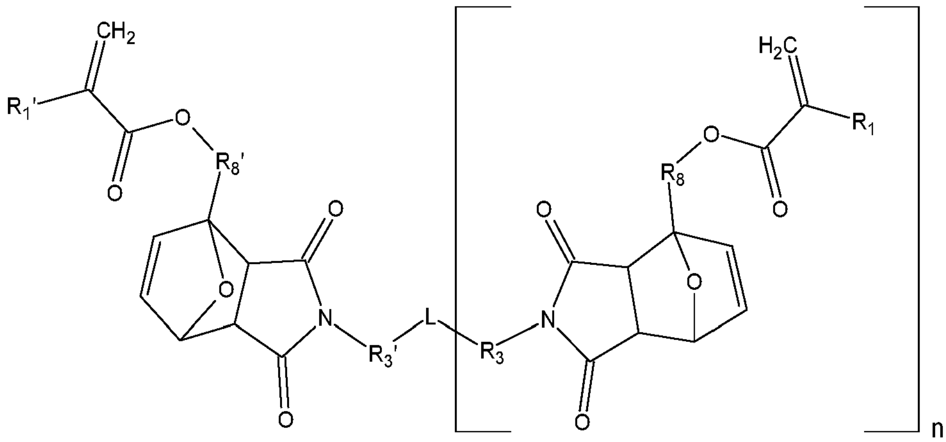

(Ic)

dans laquelle :
les groupements $R_8$ et $R_8$' sont identiques ou différents et représentent une simple liaison ou un groupe alkyle à chaine linéaire, ou ramifiée contenant jusqu'à 6 atomes de carbone.

5. Composition liquide selon l'une des revendications 1 **caractérisé en ce que** le composé réticulant est choisi dans le groupe constitué des composés de formule (Ie)

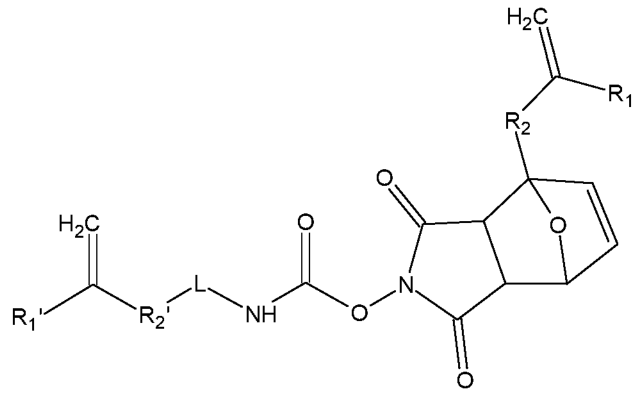

(Ie)

6. Composition liquide selon l'une des revendications 1 **caractérisé en ce que** le composé réticulant est choisi dans le groupe constitué des composés de formule (If)

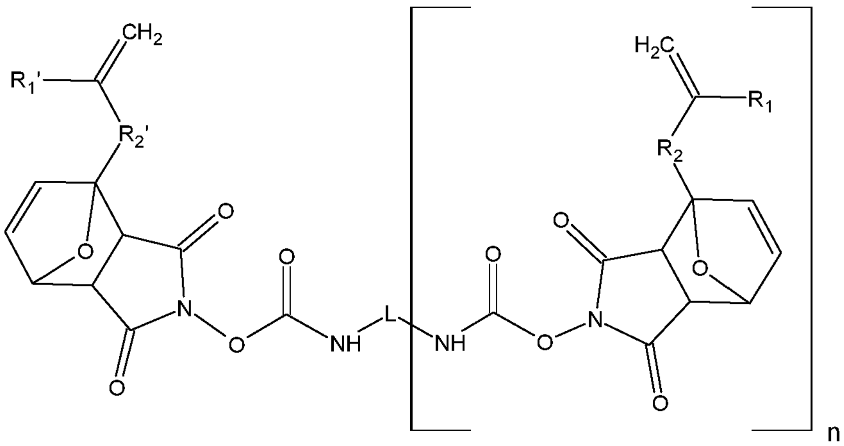

(If)

7. Composition liquide selon l'une quelconque des revendications 1 à 6, **caractérisée en ce que** la quantité de composé réticulant dans la composition est inférieure à 5 phr, par rapport à la somme du monomère (méth)acrylique (M1) et du polymère (méth)acrylique (P1).

8. Composition liquide selon l'une quelconque des revendications 1 à 7, **caractérisée en ce que** la quantité de composés libres diénes conjugués/diénophile, aptes à former un produit d'addition Diels-Alder, est inférieure à 5 phr, de préférence inférieure à 1 phr, par rapport à la somme du monomère (méth)acrylique (M1) et du polymère (méth)acrylique (P1).

9. Procédé de préparation d'une composition liquide selon l'une quelconque des revendications 1 à 8, ledit procédé comportant une première étape de mélange d'un composé réticulant selon l'une des revendications 1 à 6, à un sirop comprenant un monomère (méth)acrylique (M1) ou un mélange de monomères (méth)acryliques, et au moins un polymère (méth)acrylique (P1).

10. Procédé de préparation d'une composition liquide selon la revendication 9, **caractérisé en ce que** la première étape correspond à la solubilisation du composé réticulant dans un sirop comprenant un monomère (méth)acrylique (M1) ou un mélange de monomères (méth)acryliques et **en ce que** ledit procédé comporte une seconde étape d'ajout d'au moins un polymère (méth)acrylique (P1) au mélange préparé dans la première étape.

11. Utilisation de la composition liquide selon l'une quelconque des revendications 1 à 8 pour fabriquer des formulations pour les arts graphiques tels que des encres ou des vernis, pour des revêtements, pour des adhésifs tels que des adhésifs structuraux, pour des peintures telles que des peintures routières, pour l'étanchéité de toits ou de sols, pour des enduits gélifiés ou des couches de finition tels que pour les marbres artificiels, pour des chevilles chimiques ou des renforts pour ciment.

**12.** Utilisation de la composition liquide selon l'une quelconque des revendications 1 à 8 pour fabriquer des pièces thermoplastiques ou fabriquer des pièces composites.

**13.** Procédé de fabrication de pièces thermoplastiques comprenant les étapes suivantes :

i) placement de la composition liquide selon l'une quelconque des revendications 1 à 8 ou préparée selon la revendication 9 dans des moyens de polymérisation, et
ii)polymérisation.

**14.** Procédé de fabrication de pièces composites comprenant les étapes suivantes :

i) imprégnation de fibres ou d'un substrat fibreux avec la composition liquide selon l'une quelconque des revendications 1 à 8 ou préparée selon la revendication 9, et
ii) polymérisation.

**15.** Matériau composite polymère comprenant une matrice (méth)acrylique thermoplastique et un substrat fibreux utilisé en tant que renforcement, dans lequel le substrat fibreux est constitué de fibres longues, ledit matériau composite étant **caractérisé en ce que** la matrice (méth)acrylique thermoplastique est obtenue après polymérisation de la composition liquide, ledit substrat fibreux étant pré-imprégné avec la composition liquide selon l'une quelconque des revendications 1 à 8.

**16.** Pièce mécanique ou élément structural constitué(e) du matériau composite selon la revendication 15.

## Patentansprüche

**1.** Flüssige Zusammensetzung, die Folgendes umfasst:

a. mindestens 5 Gew.-% und nicht mehr als 50 Gew.-% eines (Meth)Acryl-Polymers (P1),
b. zwischen 40 Gew.-% und 90 Gew.-% eines (Meth)Acryl-Monomers (M1) oder einer Mischung von (Meth)Acryl-Monomeren und
c. mehr als 0,5 phr und weniger als 10 phr, bezogen auf die Summe des (Meth) Acryl-Monomers (M1) und des (Meth)Acryl-Polymers (P1), einer vernetzenden Verbindung der Formel (I)

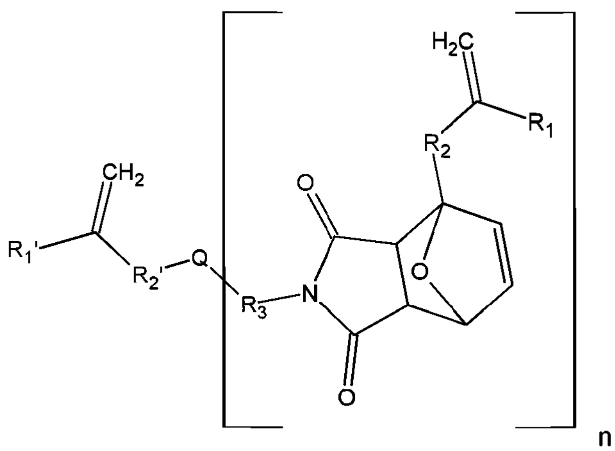

(I),

wobei:

die Gruppe Q -Z-L- darstellt, wobei:
die Gruppe Z eine Einfachbindung oder

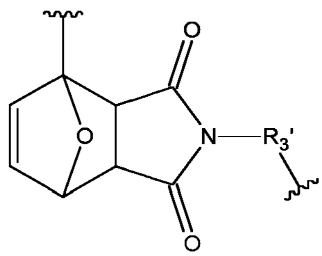

darstellt, wobei die Gruppe L eine Einfachbindung oder eine Gruppe darstellt, die ausgewählt ist aus: $-(C_1-C_6-)$Alkyl-, $-(C_2-C_6-)$Alkenyl-, $-(C_2-C_6-)$Alkinyl-, - $(CONHR_4NHCOOR_5O)_m$-, $-(O-CO-NR_6)_m$-, $-(OR_4CHOHR_5)_m$-, - $(CH_2CHOHCH_2OR_4O)_m$-, $-(OR_4)_m$-, $-((CHOR_6)CH_2O-R_4)_m$-, $-(R_4-COO-R_5)_m$-, $-NR_6$-, $-SO_2$-, $-SO_2NR_6$-, -O-, -S-, $-CONR_6$-, -Aryl-, -(Aryl-$R_4)_m$-, -(Heteroaryl-$R_4)_m$-, $-((C_3-C_8-)$Heterocyclyl-$R_4)_m$-, $-((C_3-C_{14}-)$Cycloalkyl-$R_4)_m$-, $-(C_1-C_6-)$Alkyl-$R_7(C_1-C_6-)$alkyl-, $-(C_2-C_6-)$Alkenyl-$R_7-(C_1-C_6-)$alkyl-, $-(C_1-C_6-)$Alkyl-$R_7-(C_2-C_6-)$alkenyl- oder $-(C_2-C_6-)$Alkenyl-$R_7-(C_2-C_6)$-alkenyl-,

wobei die Gruppen $R_4$ und $R_5$ gleich oder verschieden sind und eine Gruppe darstellen, die ausgewählt ist aus: einer Einfachbindung, $-(C_1-C_6)$Alkyl-, $-(C_2-C_6-)$Alkenyl-, $-NR_6$-, $-SO_2$-, $-SO_2NR_6$-, -O-, -S-, - $CONR_6$-, -Aryl-, -(Aryl-$R_6)_m$-, -(Heteroaryl-$R_6)_m$-, $-((C_3-C_8-)$Heterocyclyl)-$R_6)_m$-, $-((C_3-C_{14}-)$Cycloalkyl-$R_6)_m$-aryl-, -Heteroaryl-, $-(C_3-C_8-)$Heterocyclyl-, $-(C_3-C_{14}-)$Cycloalkyl-, $-(C_1-C_6-)$Alkyl-OCO- oder $-CH_2-(CHOR_3)CH_2O-(C_1-C_6-)$Alkyl-;
wobei $R_6$ eine Gruppe darstellt, die ausgewählt ist aus: einem Wasserstoffatom, $-(C_1-C_6-)$Alkyl, $-(C_2-C_6-)$Alkenyl, $-NH_2$, -COOH, $-SO_2H$, -OH, -SH, -Aryl, - Heteroaryl, $-(C_3-C_8-)$Heterocyclyl oder $-(C_3-C_{14}-)$Cycloalkyl;
wobei $R_7$ eine Gruppe darstellt, die ausgewählt ist aus: einer Einfachbindung, $-(CONHR_4NHCOOR_5O)_m$-, $-(O-CO-NR_4)_m$-, $-(COR_4)_m$-, $-(OR_4)_m$-, $-(CH_2CHOHCH_2OR_4O)_m$-, - $((CHOR_4)CH_2O-R_5)_m$-, $-(R_4-COO-R_5)_m$-, $-NR_6$-, $-SO_2$-, $-SO_2NR_6$-, -O-, -S-, $-CONR_6$-, -Aryl-, -(Aryl-$R_4)_m$-, -(Heteroaryl-$R_4)_m$-, $-((C_3-C_8-)$Heterocyclyl-)$R_4)_m$-, $-((C_3-C_{14}-)$Cycloalkyl-$R_4)_m$-, $-(C_1-C_6-)$Alkyl-OCO- oder $-CH_2-(CHOR_3)CH_2O-(C_1-C_6-)$Alkyl-;

wobei die Gruppen $R_1$ und $R_1$' gleich oder verschieden sind und ein Wasserstoffatom oder eine Alkylgruppe mit einer linearen oder verzweigten Kette darstellen, die bis zu 6 Kohlenstoffatome enthält;
die Gruppen $R_2$ und $R_2$' gleich oder verschieden sind und eine Einfachbindung oder eine Gruppe darstellen, die ausgewählt ist aus: $-(C_1-C_6-)$Alkyl-, $-(C_2-C_6-)$Alkenyl-, - COO- oder COO-$(C_1-C_6-)$Alkyl-, vorzugsweise -COO- oder COO-$(C_1-C_6-)$Alkyl-;
die Gruppen $R_3$ und $R_3$' gleich oder verschieden sind und -NHCOO- darstellen;
wobei n, das die Zahl der Repetiereinheiten darstellt, zwischen 1 und 10 beträgt,
wobei m, das die Zahl der Repetiereinheiten darstellt, zwischen 1 und 20, beispielsweise zwischen 1 und 10, beträgt.

2. Flüssige Zusammensetzung nach Anspruch 1, **dadurch gekennzeichnet, dass** die vernetzende Verbindung ausgewählt ist aus der Gruppe bestehend aus Verbindungen der Formel (Ia)

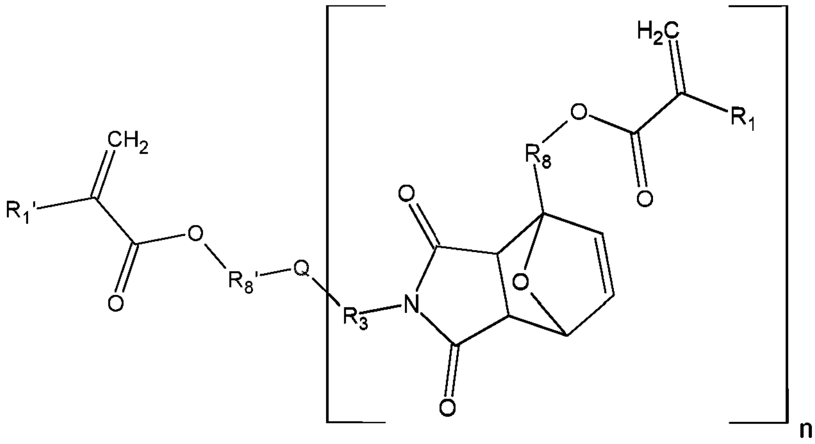

(Ia),

wobei:
die Gruppen $R_8$ und $R_8$' gleich oder verschieden sind und eine Einfachbindung oder eine Alkylgruppe mit einer linearen oder verzweigten Kette darstellen, die bis zu 6 Kohlenstoffatome enthält.

3. Flüssige Zusammensetzung nach einem der Ansprüche 1 oder 2, **dadurch gekennzeichnet, dass** die vernetzende Verbindung ausgewählt ist aus der Gruppe bestehend aus Verbindungen der Formel (Ib)

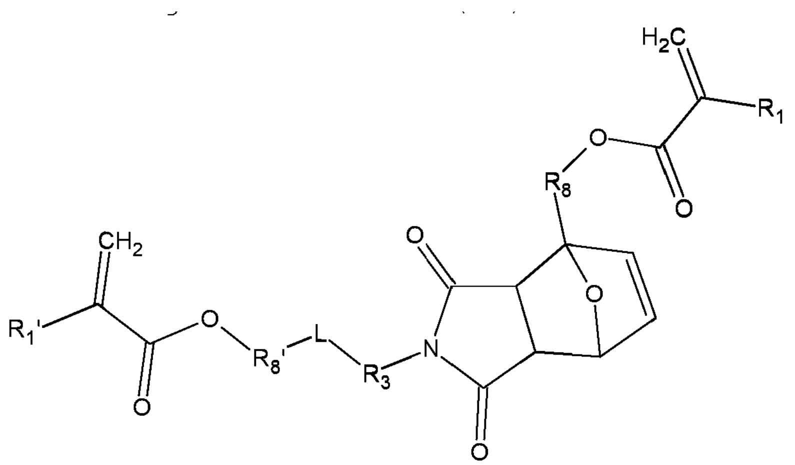

(Ib),

wobei:
die Gruppen $R_8$ und $R_8$' gleich oder verschieden sind und eine Einfachbindung oder eine Alkylgruppe mit einer linearen oder verzweigten Kette darstellen, die bis zu 6 Kohlenstoffatome enthält.

4. Flüssige Zusammensetzung nach einem der Ansprüche 1 oder 2, **dadurch gekennzeichnet, dass** die vernetzende Verbindung ausgewählt ist aus der Gruppe bestehend aus Verbindungen der Formel (Ic)

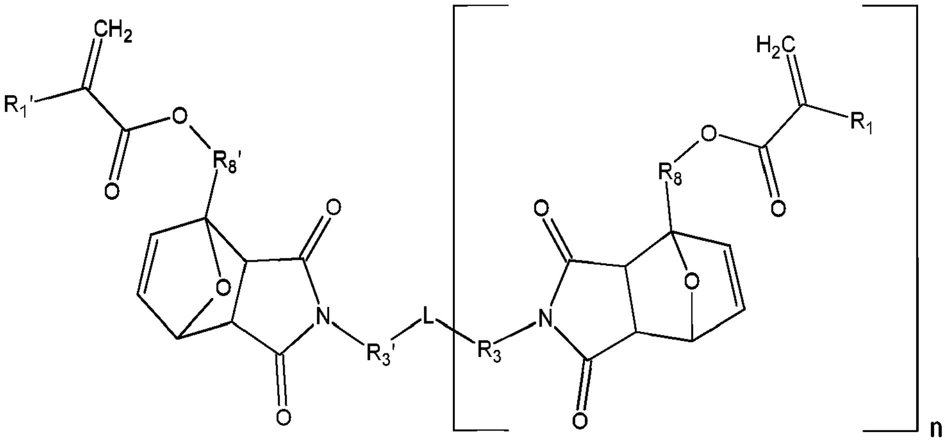

(Ic),

wobei:
die Gruppen $R_8$ und $R_8$' gleich oder verschieden sind und eine Einfachbindung oder eine Alkylgruppe mit einer linearen oder verzweigten Kette darstellen, die bis zu 6 Kohlenstoffatome enthält.

**5.** Flüssige Zusammensetzung nach einem der Ansprüche 1, **dadurch gekennzeichnet, dass** die vernetzende Verbindung ausgewählt ist aus der Gruppe bestehend aus Verbindungen der Formel (Ie)

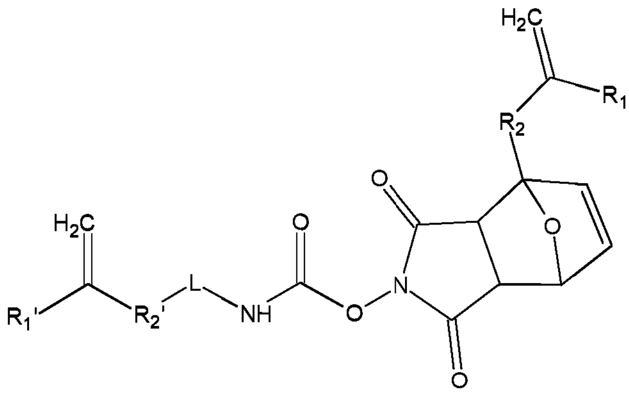

(Ie).

**6.** Flüssige Zusammensetzung nach einem der Ansprüche 1, **dadurch gekennzeichnet, dass** die vernetzende Verbindung ausgewählt ist aus der Gruppe bestehend aus Verbindungen der Formel (If)

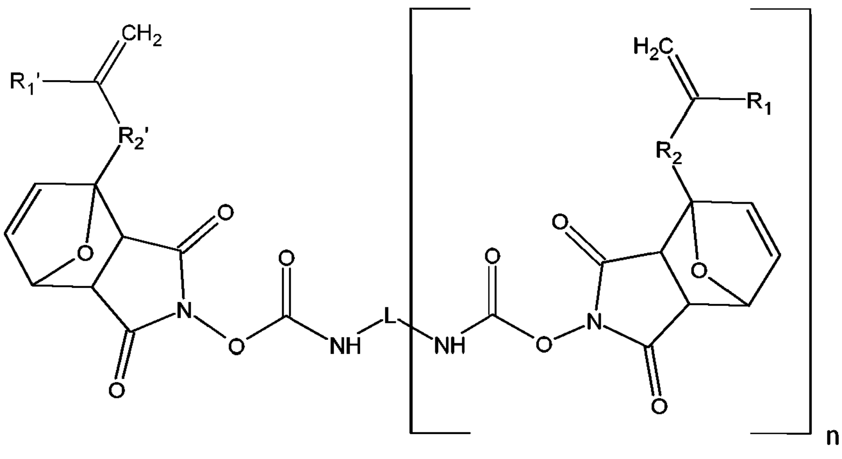

(If).

7. Flüssige Zusammensetzung nach einem der Ansprüche 1 bis 6, **dadurch gekennzeichnet, dass** die Menge der vernetzenden Verbindung in der Zusammensetzung weniger als 5 phr beträgt, bezogen auf die Summe des (Meth)Acryl-Monomers (M1) und des (Meth)Acryl-Polymers (P1).

8. Flüssige Zusammensetzung nach einem der Ansprüche 1 bis 7, **dadurch gekennzeichnet, dass** die Menge von freien konjugierten Dien-/dienophilen Verbindungen, die zur Bildung eines Diels-Alder-Addukts fähig sind, weniger als 5 phr, vorzugsweise weniger als 1 phr, beträgt, bezogen auf die Summe des (Meth)Acryl-Monomers (M1) und des (Meth)Acryl-Polymers (P1).

9. Verfahren zur Herstellung einer flüssigen Zusammensetzung nach einem der Ansprüche 1 bis 8, wobei das Verfahren einen ersten Schritt des Mischens einer vernetzenden Verbindung nach einem der Ansprüche 1 bis 6 mit einem Sirup umfasst, der ein (Meth)Acryl-Monomer (M1) oder eine Mischung von (Meth)Acryl-Monomeren und mindestens ein (Meth)Acryl-Polymer (P1) umfasst.

10. Verfahren zur Herstellung einer flüssigen Zusammensetzung nach Anspruch 9, **dadurch gekennzeichnet, dass** der erste Schritt der Löslichmachung der vernetzenden Verbindung in einem Sirup entspricht, der ein (Meth)Acryl-Monomer (M1) oder eine Mischung von (Meth)Acryl-Monomeren umfasst, und dadurch, dass das Verfahren einen zweiten Schritt des Zugebens mindestens eines (Meth)Acryl-Polymers (P1) zu der im ersten Schritt hergestellten Mischung umfasst.

11. Verwendung der flüssigen Zusammensetzung nach einem der Ansprüche 1 bis 8 zur Herstellung von Formulierungen für die Grafik, wie Druckfarben oder Lacke, für Beschichtungen, für Klebstoffe, wie Konstruktionsklebstoffe, für Farben, wie Straßenmarkierungsfarben, zur Abdichtung von Dächern oder Böden, für Gelschichten oder Deckschichten, wie für Kunstmarmore, für chemische Dübel oder Zementverstärkungen.

12. Verwendung der flüssigen Zusammensetzung nach einem der Ansprüche 1 bis 8 zur Herstellung von thermoplastischen Teilen oder zur Herstellung von Verbundbauteilen.

13. Verfahren zur Herstellung von thermoplastischen Teilen, das die folgenden Schritte umfasst:

    i) ein Anordnen der flüssigen Zusammensetzung nach einem der Ansprüche 1 bis 8 oder die gemäß Anspruch 9 hergestellt ist, in Polymerisationsvorrichtungen und
    ii) eine Polymerisation.

14. Verfahren zur Herstellung von Verbundbauteilen, das die folgenden Schritte umfasst:

    i) ein Tränken von Fasern oder eines faserförmigen Substrats mit der flüssigen Zusammensetzung nach einem der Ansprüche 1 bis 8 oder die gemäß Anspruch 9 hergestellt ist, und
    ii) eine Polymerisation.

15. Polymerer Verbundwerkstoff, der eine thermoplastische (Meth)Acryl-Matrix und ein als Verstärkung verwendetes faserförmiges Substrat umfasst, wobei das faserförmige Substrat aus Langfasern besteht, wobei der Verbundwerkstoff **dadurch gekennzeichnet ist, dass** die thermoplastische (Meth)Acryl-Matrix nach einer Polymerisation der flüssigen Zusammensetzung erhalten wird, wobei das faserförmige Substrat mit der flüssigen Zusammensetzung nach einem der Ansprüche 1 bis 8 vorgetränkt ist.

16. Mechanisches Teil oder Bauelement, das aus dem Verbundwerkstoff nach Anspruch 15 besteht.

## Claims

1. Liquid composition comprising:

    a. at least 5% by weight and not more than 50% by weight of a (meth)acrylic polymer (P1),
    b. between 40% and 90% by weight of a (meth) acrylic monomer (M1) or a blend of (meth)acrylic monomers, and
    c. greater than 0.5 phr and less than 10 phr, relative to the sum of the (meth)acrylic monomer (M1) and of the (meth)acrylic polymer (P1), of a crosslinking compound of formula (I)

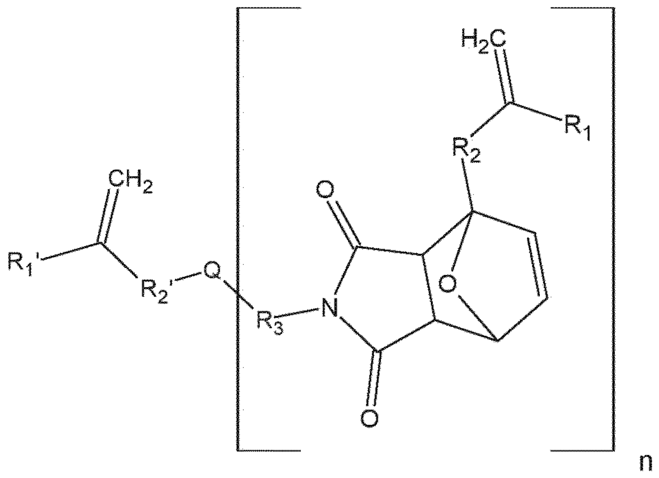

(I)

in which:

the group Q represents -Z-L- in which:

the group Z represents a single bond or

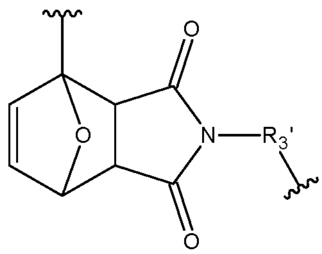

,

the group L represents a single bond or a group selected from: $-(C_1-C_6)$alkyl-, $-(C_2-C_6)$alkenyl-, $-(C_2-C_6)$alkynyl-, $-(CONHR_4NHCOOR_5O)_m-$, $-(O-CO-NR_6)_m-$, $-(OR_4CHOHR_5)_m-$, - $(CH_2CHOHCH_2OR_4O)_m-$, $-(OR_4)_m-$, $-((CHOR_6)CH_2O-R_4)_m-$, $-(R_4-COO-R_5)_m-$, $-NR_6-$, $-SO_2-$, $-SO_2NR_6-$, -O-, -S-, $-CONR_6-$, -aryl-, -(aryl-$R_4)_m-$, -(heteroaryl-$R_4)_m-$, $-((C_3-C_8)$heterocyclyl-$R_4)_m-$, $-((C_3-C_{14})$cycloalkyl-$R_4)_m-$, $-(C_1-C_6)$alkyl-$R_7-(C_1-C_6)$alkyl-, $-(C_2-C_6)$alkenyl-$R_7-(C_1-C_6)$alkyl-, $-(C_1-C_6)$alkyl-$R_7-(C_2-C_6)$alkenyl-, or $-(C_2-C_6)$alkenyl-$R_7-(C_2-C_6)$alkenyl-, in which the groups $R_4$ and $R_5$ are identical or different and represent a group selected from: single bond, $-(C_1-C_6)$alkyl-, $-(C_2-C_6)$alkenyl-, $-NR_6-$, $-SO_2-$, $-SO_2NR_6-$, -O-, -S-, $-CONR_6-$, -aryl-, -(aryl-$R_6)_m-$, -(heteroaryl-$R_6)_m-$, - $((C_3-C_8)$heterocyclyl)-$R_6)_m-$, $-((C_3-C_{14})$cycloalkyl-$R_6)_m$-aryl-, -heteroaryl-, $-(C_3-C_8)$heterocyclyl-, $-(C_3-C_{14})$cycloalkyl-, $-(C_1-C_6)$alkyl-OCO-, or $-CH_2-(CHOR_3)CH_2O-(C_1-C_6)$alkyl-;

in which $R_6$ represents a group selected from: hydrogen atom, - $(C_1-C_6)$alkyl, - $(C_2-C_6)$alkenyl, $-NH_2$, -COOH, $-SO_2H$, -OH, -SH, -aryl, -heteroaryl, $-(C_3-C_8)$heterocyclyl, or - $(C_3-C_{14})$cycloalkyl;

in which $R_7$ represents a group selected from: single bond, $-(CONHR_4NHCOOR_5O)_m-$, $-(O-CO-NR_4)_m-$, $-(COR_4)_m-$, $-(OR_4)_m-$ , $-(CH_2CHOHCH_2OR_4O)_m-$, $-((CHOR_4)CH_2O-R_5)_m-$, $-(R_4-COO-R_5)_m-$, - $NR_6-$, $-SO_2-$, $-SO_2NR_6-$, -O-, -S-, $-CONR_6-$, -aryl-, -(aryl-$R_4)_m-$, -(heteroaryl-$R_4)_m-$, $-((C_3-C_8)$heterocyclyl-$R_4)_m-$, - $((C_3-C_{14})$cycloalkyl-$R_4)_m-$, $-(C_1-C_6)$alkyl-OCO-, or $-CH_2-(CHOR_3)CH_2O-(C_1-C_6)$alkyl-;

the groups $R_1$ and $R_1$' are identical or different and represent a hydrogen atom or an alkyl group bearing a linear or branched chain containing up to 6 carbon atoms;

the groups $R_2$ and $R_2$' are identical or different and represent a single bond or a group selected from: $-(C_1-C_6)$alkyl-, $-(C_2-C_6)$alkenyl-, -COO-, or COO-$(C_1-C_6)$alkyl-, preferably -COO- or COO-$(C_1-C_6)$alkyl-;

the groups $R_3$ and $R_3$' are identical or different and represent -NHCOO-;

with n representing the number of repeating units, between 1 and 10,

with m representing the number of repeating units, between 1 and 20, for example between 1 and 10.

2. Liquid composition according to Claim 1, **characterized in that** the crosslinking compound is chosen from the group constituted of the compounds of formula (Ia)

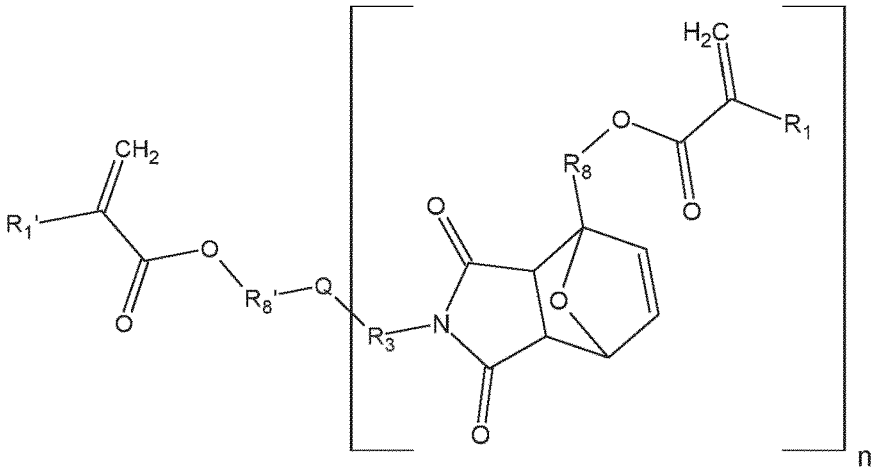

(Ia)

in which:

the groups $R_8$ and $R_8$' are identical or different and represent a single bond or an alkyl group bearing a linear or branched chain containing up to 6 carbon atoms.

3. Liquid composition according to either of Claims 1 and 2, **characterized in that** the crosslinking compound is chosen from the group constituted of the compounds of formula (Ib)

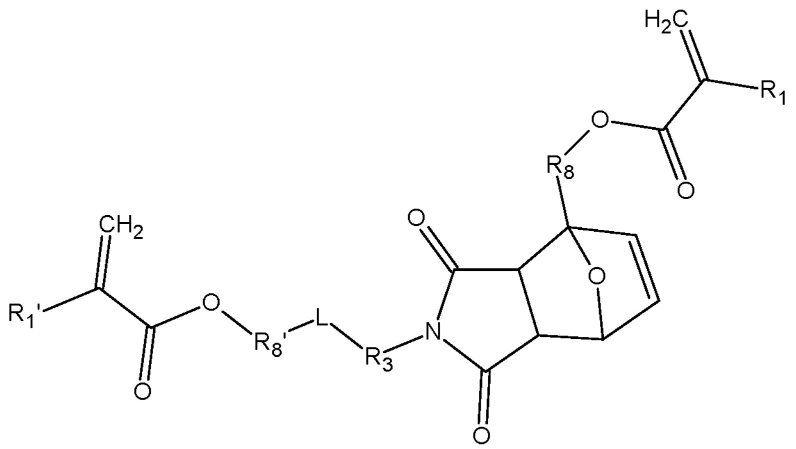

(Ib)

in which:

the groups $R_8$ and $R_8$' are identical or different and represent a single bond or an alkyl group bearing a linear or branched chain containing up to 6 carbon atoms.

4. Liquid composition according to either of Claims 1 and 2, **characterized in that** the crosslinking compound is chosen from the group constituted of the compounds of formula (Ic)

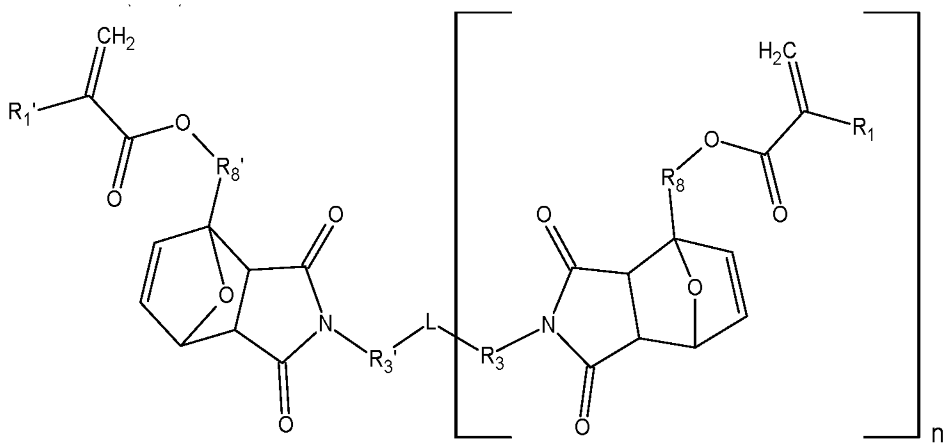

(Ic)

in which:
the groups $R_8$ and $R_8$' are identical or different and represent a single bond or an alkyl group bearing a linear or branched chain containing up to 6 carbon atoms.

**5.** Liquid composition according to one of Claims 1, **characterized in that** the crosslinking compound is chosen from the group constituted of the compounds of formula (Ie)

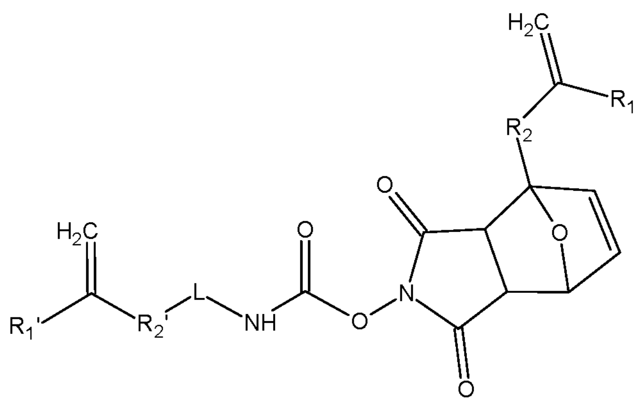

(Ie)

**6.** Liquid composition according to one of Claims 1, **characterized in that** the crosslinking compound is chosen from the group constituted of the compounds of formula (If)

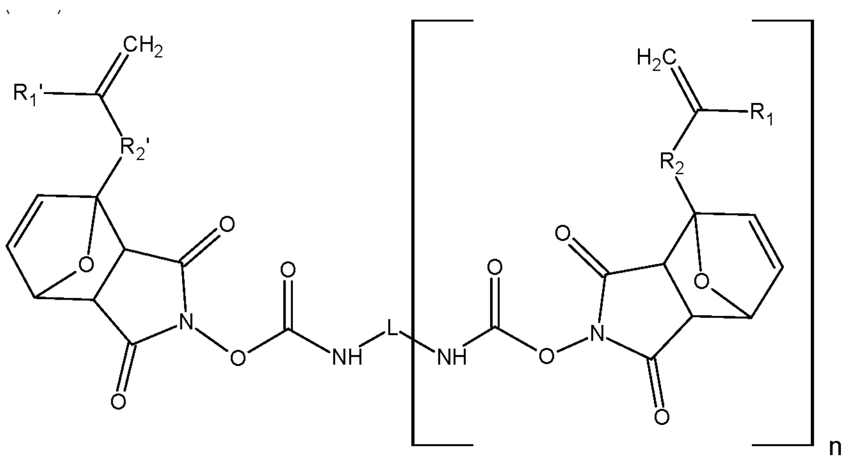

(If)

**7.** Liquid composition according to any one of Claims 1 to 6, **characterized in that** the amount of crosslinking compound in the composition is less than 5 phr, relative to the sum of the (meth)acrylic monomer (M1) and of the (meth)acrylic

polymer (P1).

**8.** Liquid composition according to any one of Claims 1 to 7, **characterized in that** the amount of free conjugated diene/dienophile compounds, capable of forming a Diels-Alder adduct, is less than 5 phr, preferably less than 1 phr, relative to the sum of the (meth) acrylic monomer (M1) and of the (meth)acrylic polymer (P1).

**9.** Process for preparing a liquid composition according to any one of Claims 1 to 8, said process including a first step of mixing a crosslinking compound according to one of Claims 1 to 6 with a syrup comprising a (meth)acrylic monomer (M1) or a blend of (meth)acrylic monomers, and at least one (meth)acrylic polymer (P1).

**10.** Process for preparing a liquid composition according to Claim 9, **characterized in that** the first step corresponds to the dissolution of the crosslinking compound in a syrup comprising a (meth)acrylic monomer (M1) or a blend of (meth) acrylic monomers and **in that** said process includes a second step of adding at least one (meth)acrylic polymer (P1) to the mixture prepared in the first step.

**11.** Use of the liquid composition according to any one of Claims 1 to 8 for manufacturing formulations for the graphic arts such as inks or varnishes, for coatings, for adhesives such as structural adhesives, for paints such as road paints, for roof or floor sealings, for gelcoats or topcoats such as for artificial marbles, for chemical dowels or cement reinforcements.

**12.** Use of the liquid composition according to any one of Claims 1 to 8 for manufacturing thermoplastic parts or manufacturing composite parts.

**13.** Process for manufacturing thermoplastic parts, comprising the following steps:

i) placing the liquid composition according to any one of Claims 1 to 8 or prepared according to Claim 9 in polymerization means, and
ii) polymerizing.

**14.** Process for manufacturing composite parts, comprising the following steps:

i) impregnating fibres or a fibrous substrate with the liquid composition according to any one of Claims 1 to 8 or prepared according to Claim 9, and
ii) polymerizing.

**15.** Polymeric composite material comprising a thermoplastic (meth)acrylic matrix and a fibrous substrate used as reinforcement, in which the fibrous substrate is constituted of long fibres, said composite material being **characterized in that** the thermoplastic (meth)acrylic matrix is obtained after polymerization of the liquid composition, said fibrous substrate being preimpregnated with the liquid composition according to any one of Claims 1 to 8.

**16.** Mechanical part or structural element constituted of the composite material according to Claim 15.

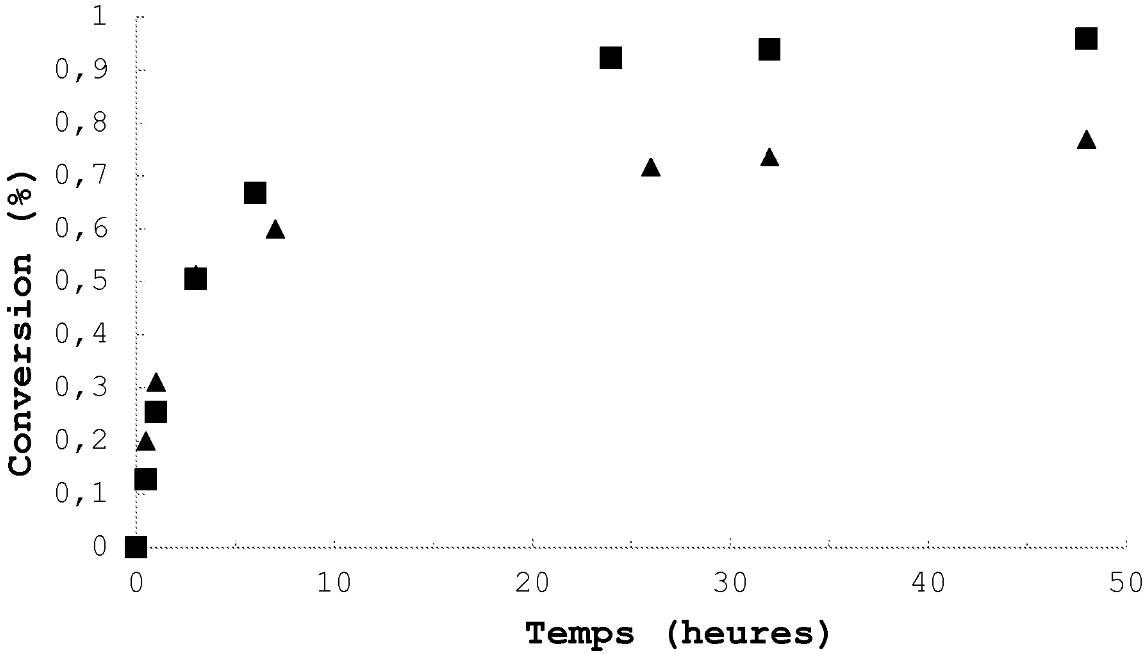

Conversion (%)
1
0,9
0,8
0,7
0,6
0,5
0,4
0,3
0,2
0,1
0
0
10
20
30
40
50
Temps (heures)


FIG. 1

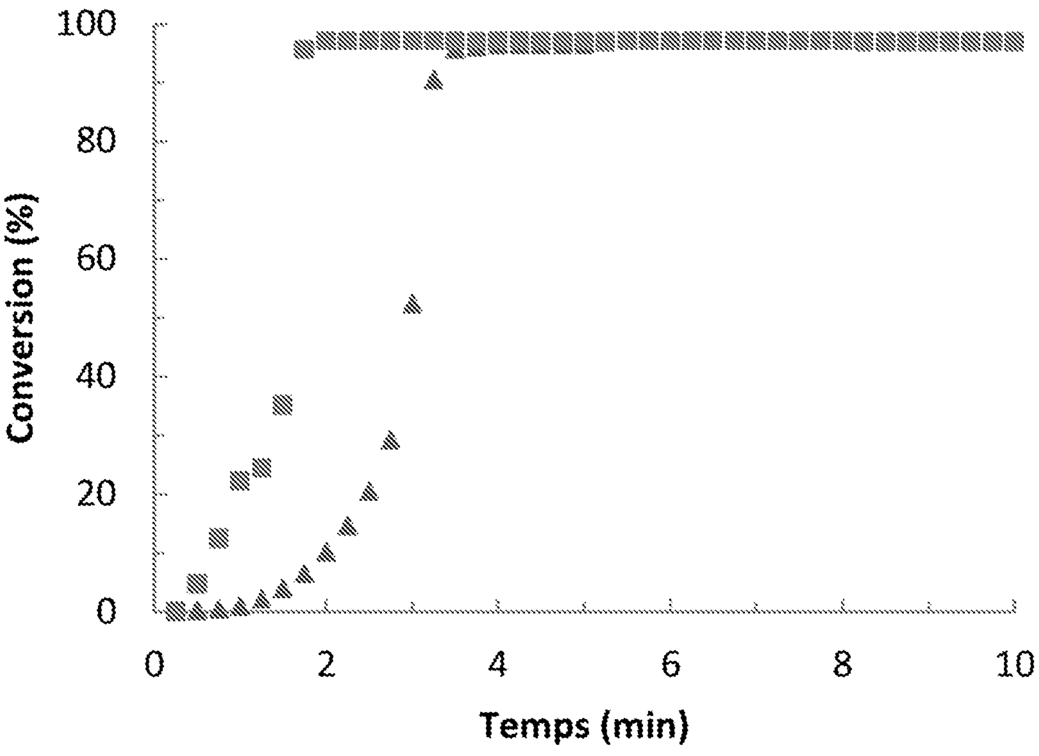

Conversion (%)
100
80
60
40
20
0
0
2
4
6
8
10
Temps (min)


FIG. 2

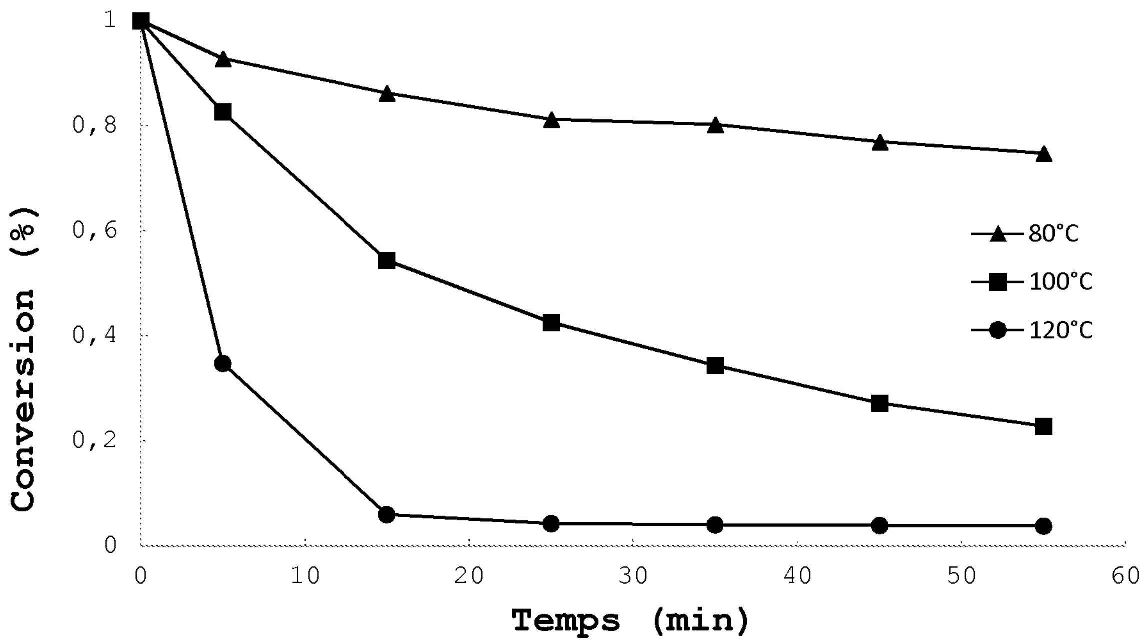
1
0,8
0,6
0,4
0,2
0
0
10
20
30
40
50
60
Conversion (%)
Temps (min)
80°C
100°C
120°C

FIG. 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2013056845 A
- WO 2014013028 A
- WO 2014174098 A
- GB 2453112 A
- JP 2014169384 B
- JP 2005232412 B
- WO 2010033028 A